# FASCICULE DE BREVET EUROPEEN

(11) **EP 0 977 868 B1**
(45) Date de publication et mention de la délivrance du brevet: **21.07.2004**
(21) Numéro de dépôt: 99901708.0
(22) Date de dépôt: 04.02.1999
(51) Int. Cl.: C12N 15/54, C12N 1/19, C12P 33/00, C12N 9/10

(54) **SOUCHES DE LEVURE AYANT LE GENE ATF2 INTERROMPU ET LEURS APPLICATIONS**
HEFESTÄMME MIT UNTERBROCHENEM ATF2-GEN UND DEREN VERWENDUNGEN
YEAST STRAINS WITH INTERRUPTED ATF2 GENE AND USES

(30) Priorité: 05.02.1998 FR 9801329
(43) Date de publication de la demande: 09.02.2000
(73) Titulaire: Aventis Pharma S.A., 92160 Antony (FR)
(72) Inventeur: ACHSTETTER, Tilman, D-77704 Oberkirch (DE); CAUET, Gilles, F-67370 Griesheim/Souffel (FR); DEGRYSE, Eric, F-67100 Strasbourg (FR)
(86) Numéro de dépôt international: PCT/FR1999/000237
(87) Numéro de publication internationale: WO 1999/040203

(56) Documents cités:
- EP-A- 0 340 878
- EP-A- 0 574 941
- EP-A- 0 727 489
- DUPORT C ET AL: "Self-sufficient biosynthesis of pregnenolone and progesterone in engineered yeast." NATURE BIOTECHNOLOGY, vol. 16, no. 2, 1 février 1998, pages 186-189, XP002084824
- DATABASE EMBL Accession number E12032, 8 octobre 1997 NAGASAWA S ET AL: "DNA encoding Saccharomyces alcohol acetyltransferase" XP002084826 -& DATABASE WPI Section Ch, Week 9648 Derwent Publications Ltd., London, GB; Class D16, AN 96-479898 XP002084827 & JP 08 242851 A (KIRIN BREWERY KK) , 24 septembre 1996
- TAKETANI S ET AL: "Characterization of sterol-ester synthetase in Saccharomyces cerevisiae" BIOCHIMICA ET BIOPHYSICA ACTA, vol. 575, no. 1, octobre 1979, pages 148-155, XP002084825

## Description

La formation de 3-oxo-delta⁴-stéroïdes à partir de précurseurs 3β-hydroxy-delta⁵ dans la biosynthèse de toutes les classes d'hormones stéroïdes chez les mammifères est catalysée par le système enzymatique 3β-hydroxy-delta⁵-stéroïde déshydrogénase (EC 1.1.1.145) et delta⁵-delta⁴-stéroïde isomérase (EC 5.3.3.1), dénommé 3β-HSD. La 3β-HSD catalyse par exemple la transformation de la prégnénolone en progestérone, de la 17α-hydroxyprégnénolone en 17α-hydroxyprogestérone, de la déhydroépiandrostérone en delta⁴-androstènedione ou du 5-androstène-3β-17β-diol en testostérone (Simard et al., 1996).

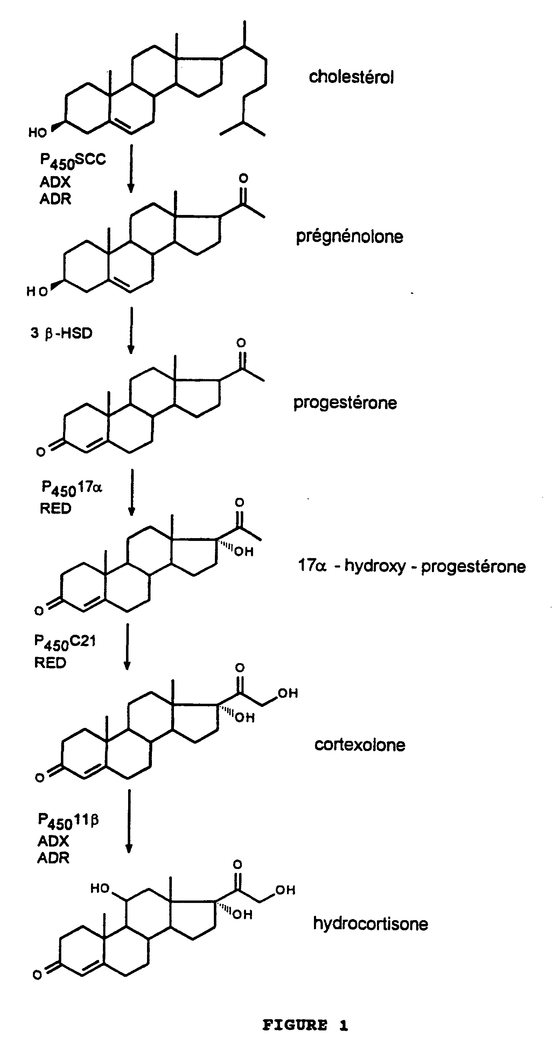

La 3β-HSD représente donc l'une des enzymes-clé de la voie de biosynthèse de l'hydrocortisone à partir du cholestérol dans le cortex adrénalien des mammifères (Figure 1).

L'utilisation de microorganismes recombinants, en particulier de levures transformées permettant l'expression hétérologue de l'un ou plusieurs des enzymes de mammifères de cette voie de biosynthèse pour produire de l'hydrocortisone ou des intermédiaires de cette biosynthèse a été décrite par exemple dans la demande de brevet européenne EP 340878, le brevet US 5,137,822, Dumas et al., 1994 et Cauet et al., 1994.

Lorsque la 3β-HSD fonctionnelle est exprimée dans la levure, les cellules de levure transformées ne convertissent pas complètement les 3β-hydroxystéroides en 3-oxostéroïdes correspondants, par exemple la prégnénolone en progestérone, mais accumulent un composé observé également dans le cas des cellules de la levure non transformée. L'identification du composé accumulé à l'ester 3β-acétique du stéroïde de départ et la caractérisation de l'enzyme ayant une activité acyl-transférase responsable de cette estérification (dénommée plus loin APAT pour "acétyl-coenzyme A prégnénolone acétyl-transférase") sont décrits dans la présente demande. D'autre part, l'accumulation d'acétate de prégnénolone par une souche de levure transformée productrice de prégnénolone a été décrite dans la demande de brevet européenne EP 727489. Ces observations permettent de considérer que l'estérification des 3β-hydroxystéroïdes produite par la levure est indésirable puisqu'elle est responsable de réactions secondaires et de sous-produits conduisant à une diminution du rendement en 3β-hydroxystéroïdes accumulés, par exemple en prégnénolone ou à une diminution du rendement de bioconversion de 3β-hydroxy-delta⁵-stéroïdes en 3-oxo-delta⁴-stéroïdes, notamment dans la production de la progestérone ou de la 17α-hydroxy-progestérone conduisant à une diminution de production ultérieure de l'hydrocortisone par la voie de biosynthèse déjà citée.

A la suite des résultats obtenus mentionnés ci-dessus, la présente invention décrit la construction de souches de levure, ayant perdu l'activité APAT indésirable, par altération du gène codant pour cette activité et dont il résulte une stabilisation des 3β-hydroxystéroïdes en présence de celles-ci. Ces souches sont donc utilisables comme souches de départ pour construire des souches recombinantes capables de transformer des 3β-hydroxystéroïdes en produits ultérieurs avec des rendements améliorés.

L'invention décrit aussi la construction de souches de levure ayant perdu l'activité APAT par altération du gène codant pour cette activité et soit exprimant la 3β-HSD ou le cytochrome P₄₅₀17α, soit coexprimant la 3β-HSD et le cytochrome P₄₅₀17α de la voie de biosynthèse de l'hydrocortisone à partir du cholestérol. Les souches exprimant par exemple la 3β-HSD permettent d'améliorer les rendements de bioconversion des 3β-hydroxy-delta⁵-stéroïdes en 3-oxo-delta⁴-stéroïdes et sont donc utilisables dans les procédés de production améliorée de l'hydrocortisone ou de ses intermédiaires dans la levure.

La présente invention a donc pour objet une souche de levure modifiée dans laquelle l'activité acétyl-CoA prégnénolone acétyltransférase (APAT) est éliminée par altération du gène codant pour cette activité et dont il résulte une stabilisation des 3β-hydroxystéroïdes.

L'altération du gène codant pour l'activité APAT peut être réalisée par exemple par insertion, délétion ou substitution d'une séquence d'ADN dans les éléments fonctionnels du gène, par exemple le promoteur ou la séquence codante pour la protéine ayant l'activité APAT. L'intégration de la séquence d'ADN ainsi altérée dans une souche hôte de levure peut être ensuite effectuée par exemple par la méthodologie de la recombinaison homologue et conduit à la génération de mutants chromosomiques de levure correspondant aux souches modifiées de l'invention dans lesquelles la disparition de l'activité APAT et la stabilisation des 3β-hydroxystéroïdes sont mises en évidence, par exemple par culture des cellules en présence de prégnénolone et par mesure de la teneur en prégnénolone en fonction du temps en suivant des conditions opératoires décrites plus loin dans la partie expérimentale.

Comme souches de levure hôtes utilisées pour l'invention, on peut citer notamment les souches de *Saccharomyces* telles que *S. cerevisiae,* les souches de *Candida* telles que *C. maltosa,* les souches de *Kluyveromyces* telles que *K. lactis* ou les souches de *Pichia* telles que *P. pastoris.*

L'invention a particulièrement pour objet une souche de levure modifiée ci-dessus dans laquelle le gène altéré est le gène *ATF2* de *S. cerevisiae* ou un homologue de celui-ci.

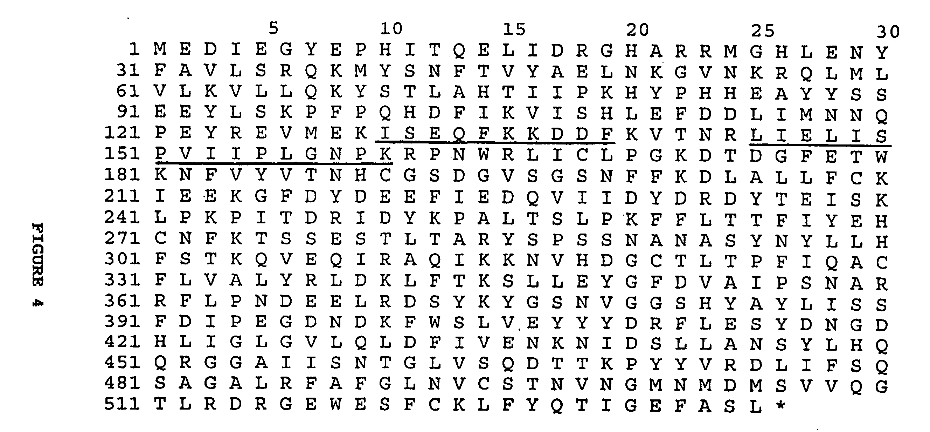

Par gène *ATF2,* on entend le gène de *S. cerevisiae* identifié dans le génome de la levure sous le locus *ATF2* ou *YGR177c,* de *"Saccharomyces* Genome Database" (SGD) ; (Cherry et al. http://genome-www.stanford.edu/*Saccharomyces*/) dont le cadre de lecture ouvert (ORF) dénommé *YGR177c* est traduit en séquence d'acides aminés dans la banque de données Mips, accessible sous le numéro d'accession S64491 (Hebling U., Hofmann B. et Delius H. (May 1996)) et dont la séquence est montrée à la figure 4. Ce gène code pour une protéine ayant l'activité APAT, comme cela est montré plus loin dans la partie expérimentale.

Par gène homologue du gène *ATF2,* on entend un gène qui code pour une protéine ayant l'activité APAT et ayant une identité de séquence d'environ 60 % et plus avec la séquence de la protéine YGR177C.

L'invention a plus particulièrement pour objet une souche de levure modifiée ci-dessus dans laquelle le gène altéré est le gène *ATF2* de *S. cerevisiae* et dénommée plus loin souche mutante *atf2*.

L'invention a spécialement pour objet une souche de levure modifiée ci-dessus, dans laquelle le gène *ATF2* est altéré par insertion d'une séquence d'ADN ayant au moins un nucléotide.

La séquence d'ADN qui est insérée dans le gène *ATF2* de façon à perdre toute activité APAT peut être, par exemple un gène de sélection auxotrophe complétant une exigence nutritionnelle de la souche hôte telle que le gène *URA3*, le gène *LEU2,* le gène *TRP1,* le gène *HIS3* ou le gène *ADE2,* par exemple un gène de sélection dominant tel qu'un gène de résistance à un antibiotique tel que G418, la phléomycine ou l'hygromycine B ou par exemple un gène rapporteur tel que le gène *βGAL.*

La séquence d'ADN qui est insérée dans le gène *ATF2* peut être aussi un bloc d'expression de levure constitué d'un promoteur et d'un terminateur de transcription, par exemple un promoteur de levure tel que *PGK, TDH3, CYC1 ou TEF1,* par exemple un terminateur de levure tel que *CYC1, TDH3, TEF1* ou *PGK.* Le bloc d'expression peut être une combinaison des éléments cités ci-dessus, par exemple le bloc *TEF1*_{*prom*}/*PGK*_{*term*}.

L'invention a plus spécialement pour objet une souche de levure modifiée ci-dessus, dans laquelle le gène *ATF2* est altéré par insertion du gène de sélection *URA3* ou du bloc d' expression *TEF1*_{*prom*}/*PGK*_{*term*}.

L'invention a particulièrement pour objet une souche de levure modifiée ci-dessus, dans laquelle le gène *ATF2* est altéré par insertion du gène de sélection *URA3*.

Les souches mutantes atf2 de l'invention, dépourvues d'activité APAT et dans lesquelles le gène *URA3* a été inséré, dénommées plus loin *atf2-*Δ::*URA3,* ont ainsi pu être sélectionnées par prototrophie à l'uracile.

L'invention a tout spécialement pour objet les souches de *S. cerevisiae* modifiées dénommées TGY156 et TGY158, dont les constructions détaillées sont données plus loin dans la partie expérimentale.

L'invention a aussi particulièrement pour objet une souche de levure modifiée ci-dessus, dans laquelle le gène ATF2 est altéré par insertion du bloc d'expression *TEF1*_{*prom*}/*PGK*_{*term*}*.* Les souches mutantes *atf2* de l'invention, dépourvues d'activité APAT et dans lesquelles le bloc d'expression *TEF1*_{*prom*}/*PGK*_{*term*} a été inséré, dénommées plus loin *atf2-Δ : : TEF1*_{*prom*}/*PGK*_{*term*}*,* ont pu être sélectionnées pour l'absence d'un gène *URA3* fonctionnel, remplacé par un bloc d'expression, par leur résistance à l'acide 5-fluoro-orotique (5-FO).

L'invention a tout spécialement pour objet la souche de *S. cerevisiae* modifiée, dénommée TGY186, dont la construction détaillée est donnée plus loin dans la partie expérimentale.

L'invention a aussi pour objet une souche de levure transformée dans laquelle l'activité acétyl-CoA prégnénolone acétyltransférase (APAT) est éliminée par altération du gène codant pour cette activité et exprimant au moins l'une des enzymes de mammifères de la voie de biosynthèse de l'hydrocortisone à partir du cholestérol choisie parmi :
- l'enzyme de clivage de la chaîne latérale du cholestérol (P₄₅₀SCC),
- la 3β-hydroxy-delta⁵-stéroïde déshydrogénase/delta⁵-delta⁴-stéroïde isomérase (3β-HSD) et
- la 17α-stéroïde hydroxylase (P₄₅₀17α).
Les souches de levure transformées de l'invention peuvent être obtenues par exemple par transformation de souches mutantes *atf2* de l'invention selon les méthodes connues, par exemple par transformation par un vecteur d'expression de P₄₅₀SCC ainsi que d'ADX et d'ADR, par un vecteur d'expression de la 3β-HSD ou par un vecteur d'expression de P₄₅₀17α. Les souches mutantes *atf2* peuvent aussi être éventuellement co-transformées, par exemple par un vecteur d'expression de la 3β-HSD et par un vecteur d'expression de P₄₅₀17α ou être transformées par un vecteur de coexpression de la 3β-HSD et de P₄₅₀17α et être utilisées par exemple dans un procédé de bioconversion de la prégnénolone en 17α-hydroxyprogestérone.

Des vecteurs construits pour l'expression de P₄₅₀SCC ainsi que d'ADX et d'ADR, de la 3β-HSD ou de P₄₅₀17α d'origine bovine ou humaine dans des souches de levure ont été décrits, par exemple par Dumas et al., 1994, dans la demande de brevet européenne EP 340878 ou dans le brevet US 5,137,822.

L'invention concerne particulièrement une souche de levure transformée ci-dessus, dans laquelle le gène altéré est le gène *ATF2* de *S. cerevisiae* ou un homologue de celui-ci. L'invention a plus particulièrement pour objet une souche de levure transformée ci-dessus, dans laquelle le gène altéré est le gène *ATF2* de *S. cerevisiae* et correspondant à une souche *atf2* transformée.

L'invention a tout particulièrement pour objet une souche de levure transformée ci-dessus, dans laquelle le gène *ATF2* est altéré par insertion d'une séquence d'ADN ayant au moins un nucléotide et a spécialement pour objet une souche de levure transformée, dans laquelle le gène *ATF2* est altéré par insertion du gène de sélection *URA3* et correspondant à une souche *atf2-Δ : : URA3* transformée.

L'altération du gène de façon à perdre toute activité APAT, le gène *ATF2* ou un homologue de celui-ci ainsi que les souches hôtes ont les significations indiquées précédemment.

L'invention concerne tout spécialement une souche de levure transformée *atf2-Δ::URA3* ci-dessus exprimant la 3β-HSD et en particulier la souche de *S. cerevisiae* transformée dénommée TGY158/pTG10862 dont une construction détaillée est décrite plus loin dans la partie expérimentale.

L'invention a aussi spécialement pour objet une souche de levure transformée ci-dessus, dans laquelle le gène *ATF2* est altéré par insertion du bloc d'expression *TEF1*_{*prom*}*lPGK*_{*term*} et correspondant à une souche *atf2-Δ:*: TEF1ₚᵣₒₘ/PGKₜₑᵣₘ transformée.

L'invention concerne aussi tout spécialement une souche transformée *atf2-Δ:: TEF1*_{*prom*}/*PGK*_{*term*} ci-dessus exprimant P₄₅₀17α et en particulier la souche de *S. cerevisiae* transformée, dénommée TGY186/pTG10435.

L'invention concerne particulièrement une souche de levure transformée *atf2-Δ:: TEF1*_{*prom*}/*PGK*_{*term*} ci-dessus coexprimant la 3β-HSD et P₄₅₀17α et tout particulièrement la souche *S. cerevisiae* transformée, dénommée TGY186/pTG10417.

L'invention a aussi pour objet un procédé d'oxydation in vivo d'un substrat choisi parmi un stérol endogène, un stérol exogène ou un stéroïde exogène dans lequel on utilise une souche de levure transformée ci-dessus que, soit l'on cultive seule lorsque la souche génère le stérol endogène, soit l'on incube avec le stérol ou le stéroïde exogène et on isole éventuellement le composé oxydé obtenu.

Par stérol endogène, on entend un stérol accumulé dans une souche de levure et qui est un substrat de l'enzyme de clivage de la chaîne latérale (P₄₅₀SCC) lorsque la levure, après transformation par exemple par un vecteur d'expression de P₄₅₀SCC, d'ADX et d'ADR, est cultivée en absence de stérol exogène. Les stérols endogènes utilisés pour la mise en oeuvre du procédé de l'invention peuvent être par exemple l'ergosta 5-ène-3-ol, l'ergosta 5,24(28)-diène-3-ol ou l'ergosta 5,22-diène-3-ol. La demande de brevet européenne EP 727489 décrit l'accumulation de tels stérols dans une souche de levure et le clivage de leur chaîne latérale dans une culture de la souche après transformation par un vecteur d'expression de P₄₅₀SCC, d'ADX et d'ADR. Une telle souche de levure, dans laquelle l'activité APAT est aussi présente, peut être modifiée au préalable pour obtenir une souche mutante *atf2* selon l'invention, puis être transformée par un vecteur d'expression de P₄₅₀SCC, d'ADX et d'ADR pour obtenir une souche mutante *atf2* transformée selon l'invention.

Par stérol exogène, on entend un stérol qui est un substrat de l'enzyme de clivage P₄₅₀SCC par incubation avec une souche de levure transformée par un vecteur d'expression de P₄₅₀SCC, d'ADX et d'ADR, par exemple le cholestérol ou le sitostérol. Une telle souche peut être par exemple une souche mutante *atf2* transformée par un vecteur d'expression de P₄₅₀SCC, d'ADX et d'ADR.

Le 3β-hydroxystéroïde obtenu par clivage de la chaîne latérale du stérol endogène ou exogène utilisé comme substrat est totalement sous forme libre, c'est-à-dire non accompagné de l'ester 3β-acétique correspondant, dans les cultures de souches *atf2* transformées exprimant P₄₅₀SCC, ADX et ADR.

Par stéroïde, on entend un stéroïde qui est un substrat de l'enzyme 3β-HSD par incubation avec une souche de levure transformée par exemple par un vecteur d'expression de la 3β-HSD, tel que la prégnénolone, la 17α-hydroxyprégnénolone ou la déhydroépiandrostérone ou un stéroïde qui est un substrat de l'enzyme P₄₅₀17α par incubation avec une souche de levure transformée par exemple par un vecteur d'expression de P₄₅₀17α, tel que la progestérone ou la prégnénolone. Une telle souche peut être par exemple une souche mutante *atf2* transformée par un vecteur d'expression de la 3β-HSD ou par un vecteur d'expression de P₄₅₀17α selon l'invention.

L'invention a particulièrement pour objet le procédé d'oxydation in vivo ci-dessus, dans lequel le substrat est un 3β-hydroxystéroïde et dans lequel on utilise une souche de levure a*tf2*-Δ : : *URA3* transformée exprimant la 3β-HSD et isole éventuellement le 3-oxo-delta⁴-stéroïde obtenu et a spécialement pour objet un procédé dans lequel le 3β-hydroxy stéroïde est choisi parmi la prégnénolone ou la 17α-hydroxyprégnénolone.

Le 3β-hydroxystéroide utilisé comme substrat est stable lorsqu'on l'incube avec une souche a*tf2-*Δ::*URA3* de l'invention transformée par un vecteur d'expression de 3β-HSD. L'invention fournit ainsi un procédé amélioré de production de 3-oxo-delta⁴-stéroïde dans une levure puisque la totalité du substrat 3β-hydroxy peut être oxydée en 3-oxo-delta⁴-stéroïde, comme cela est montré plus loin dans la partie expérimentale.

L'invention a aussi particulièrement pour objet le procédé d'oxydation in vivo ci-dessus, dans lequel le substrat est un stéroïde et dans lequel on utilise une souche de levure *atf2-Δ:: TEF1*_{*prom*}/*PGK*_{*term*} transformée exprimant P₄₅₀17α et isole éventuellement le stéroïde 17α-hydroxylé obtenu et a spécialement pour objet un procédé dans lequel le substrat stéroïde est la prégnénolone ou la progestérone.

La prégnénolone utilisée comme substrat est stable lorsqu'on l'incube avec une souche *atf2-Δ : : TEF1*_{*prom*}/*PGK*_{*term*} transformée par un vecteur d'expression de P₄₅₀17α.

L'invention fournit donc aussi un procédé amélioré de production de 17α-hydroxystéroïdes à partir de 3β-hydroxystéroides puisque la totalité du substrat 3β-hydroxy peut être 17α-hydroxylée.

L'invention a aussi particulièrement pour objet le procédé d'oxydation in vivo ci-dessus, dans lequel le substrat est un 3β-hydroxy stéroïde et dans lequel on utilise une souche de levure *atf2- Δ : : TEF1*_{*prom*}/*PGK*_{*term*} transformée coexprimant la 3β-HSD et P450-17α et isole éventuellement le 3-oxo-delta⁴-stéroïde 17α-hydroxylé obtenu. L'invention a spécialement pour objet le procédé ci-dessus dans lequel le substrat stéroïde est la prégnénolone.

Les souches de levure mutantes *atf2* transformées et le procédé de l'invention permettent de prévoir leur utilisation avantageuse dans la production améliorée de l'hydrocortisone ou de ses intermédiaires dans la levure.

Des exemples de construction de souches de l'invention et de mise en oeuvre du procédé de l'invention sont décrits plus loin dans la partie expérimentale.

### Matériels et méthodes générales

### 1. Souches et milieux

Les souches de *S. cerevisiae* utilisées pour la réalisation de l'invention sont la souche TGY73.4 (*MATα, URA3-Δ5, pra1-1, prb1-1, prc1-1, cps1-3, his*) dérivée isogénique *Leu*^{*+*} de cl3ABYS86 décrite par Achstetter et al., 1992 et la souche Fy1679 (*MATa, URA3-52, trp1-Δ63, leu2-Δ1, his3-Δ200, fen1, GAL*) décrite par Thierry et al., 1990. La croissance des souches est effectuée sur milieu complet YPD (Difco Laboratories) contenant 2 % de glucose à 28°C selon les conditions décrites par F. Sherman, 1991.

Pour la transformation de *S. cerevisiae,* les cellules sont rendues compétentes par la méthode à l'acétate de lithium (Ito et al, 1983). Les levures sont cultivées en routine sur un milieu minimum synthétique SD contenant 2 % de glucose (F. Sherman, 1991) avec addition des besoins nutritionnels à la concentration de 100 µg/ml.

La souche *E. coli* BJ5183 (D. Hanahan, 1983) a été utilisée pour la recombinaison in vivo et la souche *E. coli* C600, hsdR (Hubacek et al., 1970) a été utilisée comme souche receveuse pour les réactions classiques de ligation.

### 2. Manipulation de l'ADN et recombinaison in vivo dans E. coli

Les méthodes générales de biologie moléculaire utilisées sont décrites par Sambrook et al., 1989. La méthode pour la recombinaison in vivo a été décrite par E. Degryse, 1995 et E. Degryse, 1996.

### 3. Test de l'activité enzymatique APAT

L'activité acétyltransférase de l'APAT a été déterminée par mesure de l'incorporation de [³H] acétate dans la prégnénolone à partir de [³H]acétyl-CoA (New England Nuclear). Le milieu de réaction (500 µl) contient du [³H]acétyl-CoA (20 µM, 25 Ci/mole) et de la prégnénolone (Sigma) (30 µM). La prégnénolone est ajoutée en solution dans 2 µl de mélange tyloxapol (Sigma)/éthanol (1:1) dans un tampon phosphate de potassium (20mM) à pH 7,0. Après incubation pendant 15 minutes à 30°C, la réaction est stoppée par addition de 2 ml de dichlorométhane.

Les stéroïdes sont extraits avec du dichlorométhane, puis séparés par chromatographie liquide à haute performance en phase inverse (noté plus loin RP-HPLC) dans des conditions d'élution isocratiques avec de l'acétonitrile sur une colonne Ultrasphère ODS (Beckman) à 45°C sur un chromatographe HP 1090 (Hewlett Packard) couplé à un radiodétecteur FLO-One 500 (Packard) qui permet de mesurer la quantité de [³H] acétate de prégnénolone formée.

Une unité APAT est définie comme la quantité d'enzyme qui produit 1 nmole d'acétate de prégnénolone par minute à 30°C, dans les conditions décrites ci-dessus.

### 4. Détermination de la concentration en protéine

La concentration en protéine a été mesurée en utilisant la trousse "protein assay kit" (Bio-Rad) avec de l'albumine de sérum de boeuf comme standard.

Les figures ci-annexées illustrent certains aspects de l'invention.

La figure 1 représente la voie de biosynthèse de l'hydrocortisone à partir du cholestérol, chez les mammifères.

Les figures 2A et 2B illustrent la bioconversion de la prégnénolone en acétate de prégnénolone chez *S. cerevisiae.* L'analyse est faite par RP-HPLC à 205 nm :

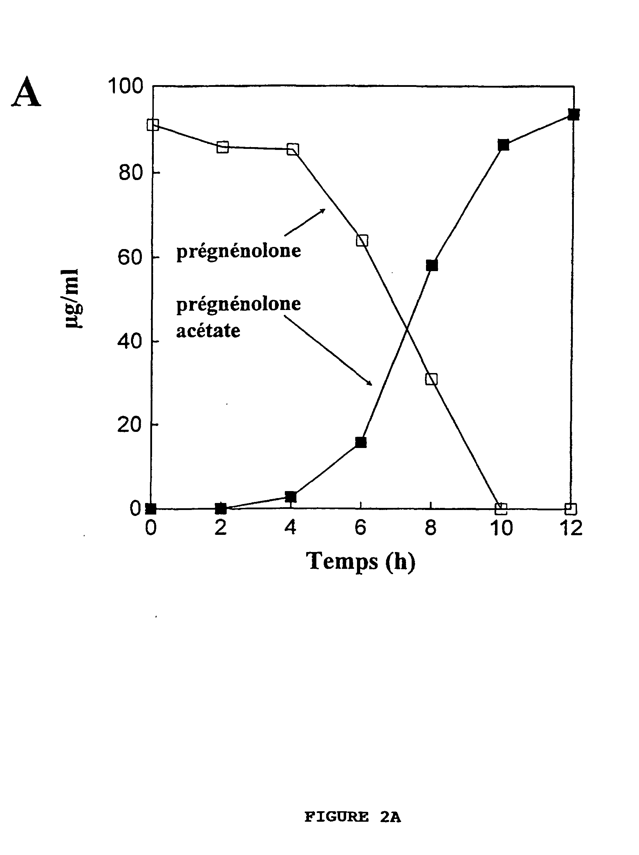

La figure 2A montre la cinétique de formation de l'acétate de prégnénolone et de disparition de la prégnénolone à des intervalles de temps sur 12 h d'incubation.

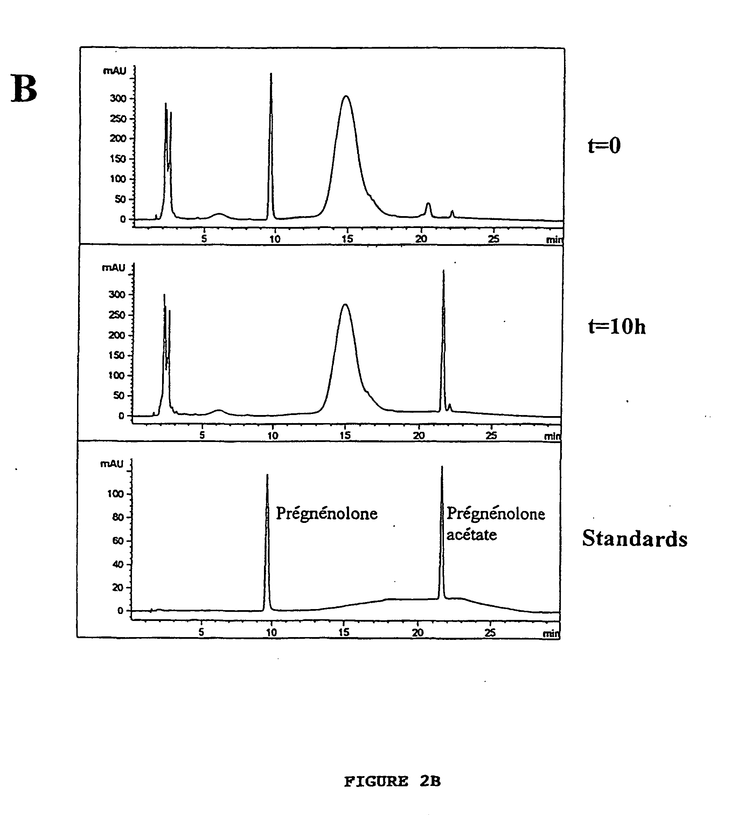

La figure 2B montre le profil en stéroïdes à t=0 et à t=10 h par rapport à un profil de standards de prégnénolone et d'acétate de prégnénolone.

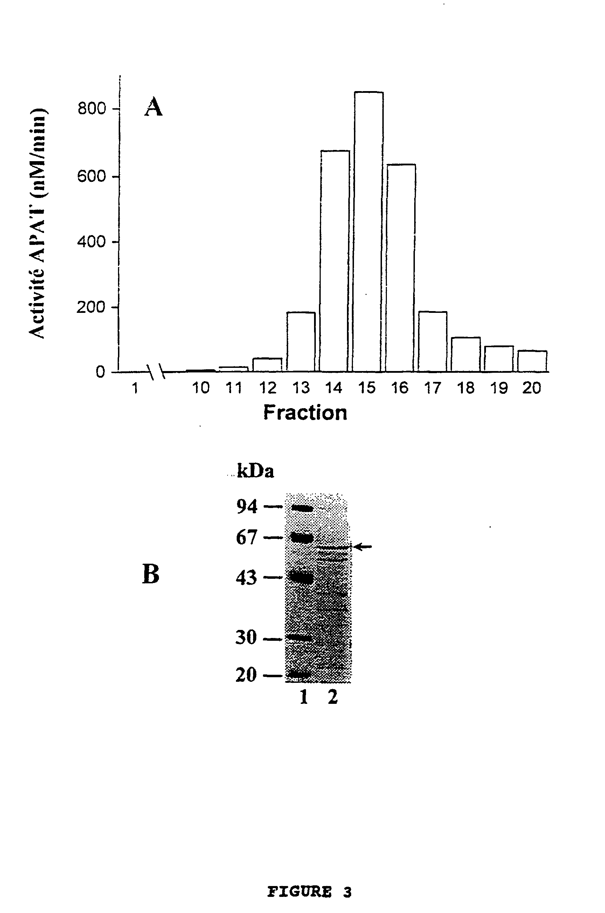

La figure 3 illustre la purification de l'APAT par chromatographie sur MonoP HR 5/20 :
(A) montre le profil de l'activité APAT présente dans les fractions 10 à 20.
(B) montre l'analyse par SDS-PAGE des fractions 14, 15 et 16 réunies et concentrées par coloration au bleu de Coomassie (ligne 2) en présence de marqueurs de poids moléculaire (ligne 1). La flèche indique la bande de PM apparent de 62kDa.

La figure 4 représente la séquence en acides aminés en code à une lettre de la protéine YGR177c. Les peptides séquencés à partir de la protéine APAT purifiée et digérée par la trypsine sont soulignés.

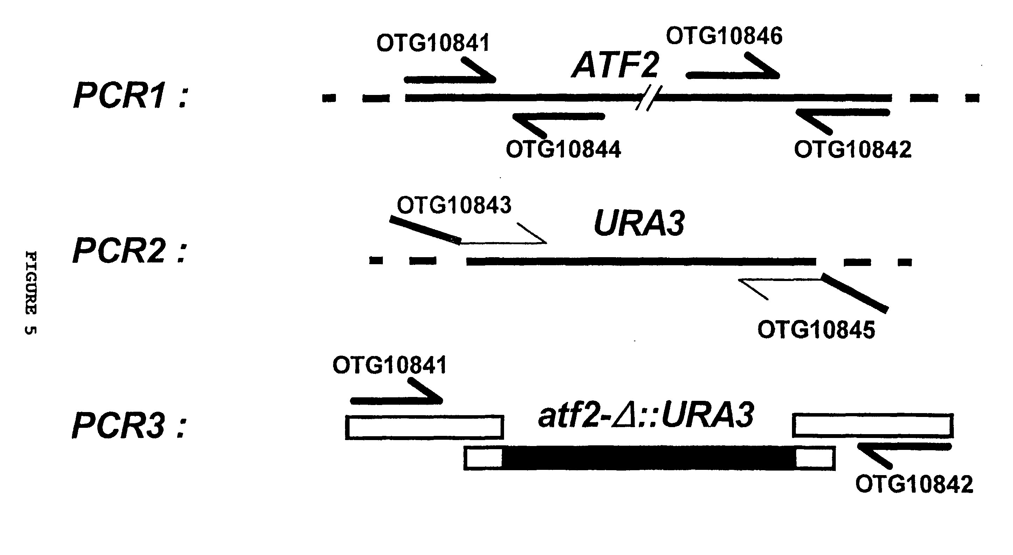

La figure 5 représente la stratégie d'interruption du gène *ATF2* par association au gène *URA3* par la méthode de double fusion PCR. Les barres vides et les barres pleines représentent les séquences d'*ATF2* et d'*URA3* respectivement.

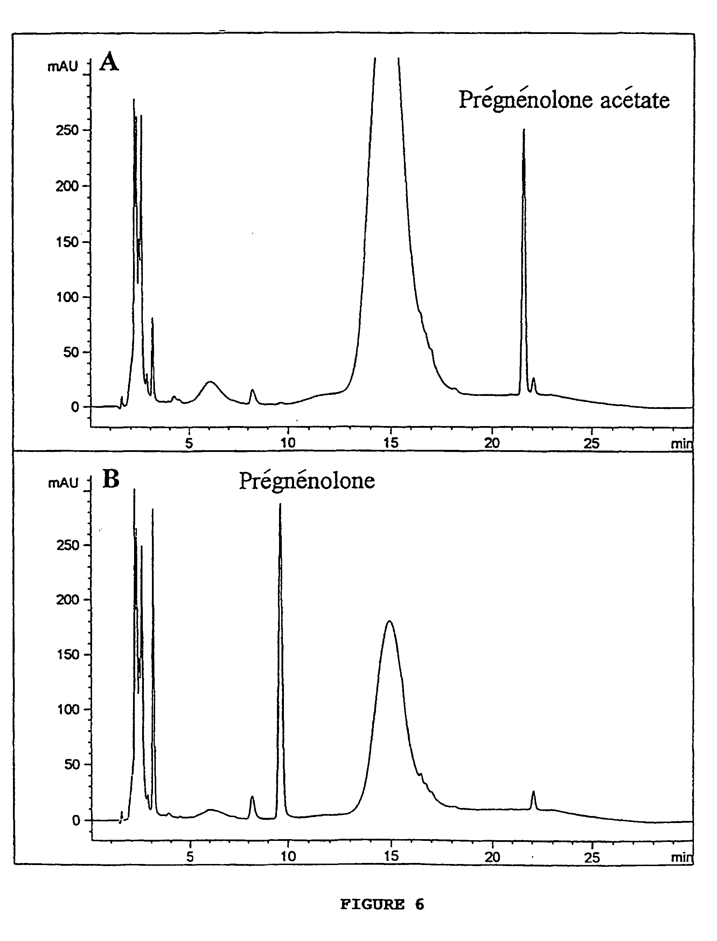

La figure 6 illustre l'effet de l'interruption du gène *ATF2* dans *S. cerevisiae* sur l'acétylation de la prégnénolone. La présence de prégnénolone acétate est mise en évidence par RP-HPLC à 205 nm à partir de cultures de 16 h de la souche parente TGY73.4 (A) ou de la souche mutante TGY158 (B).

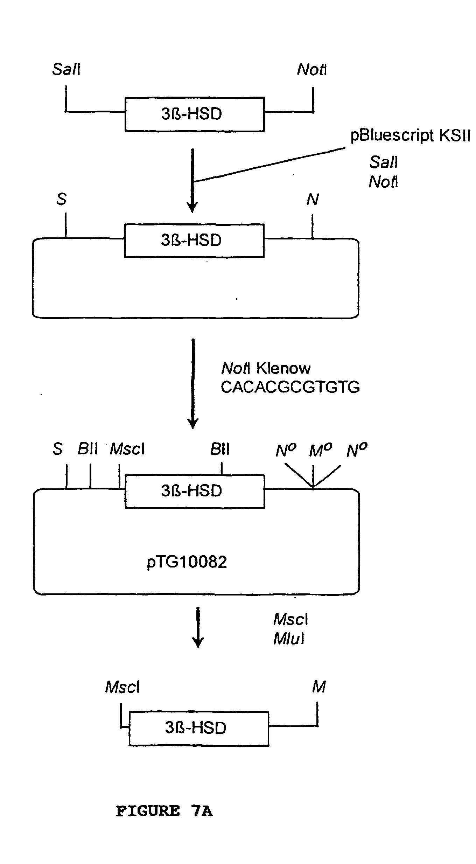

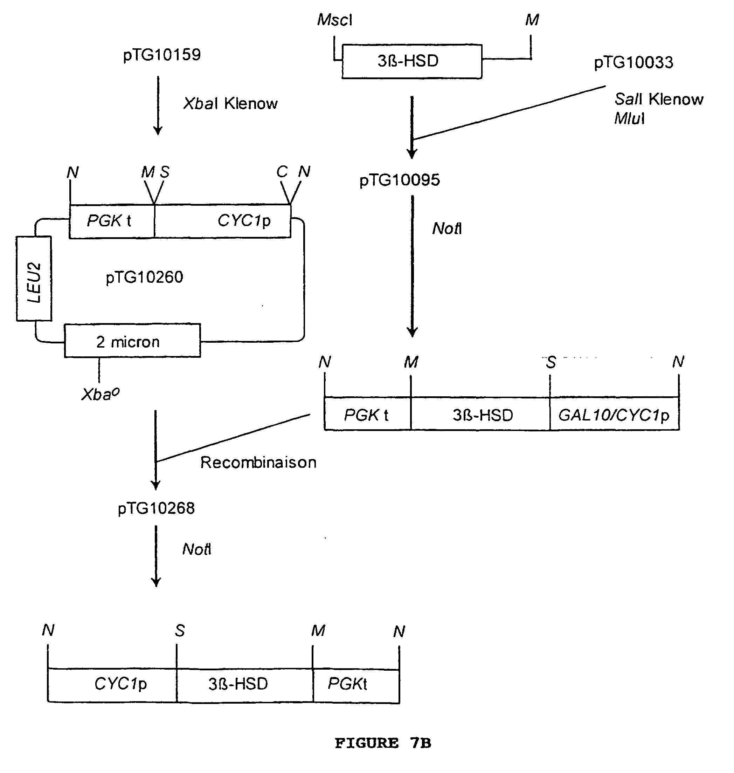

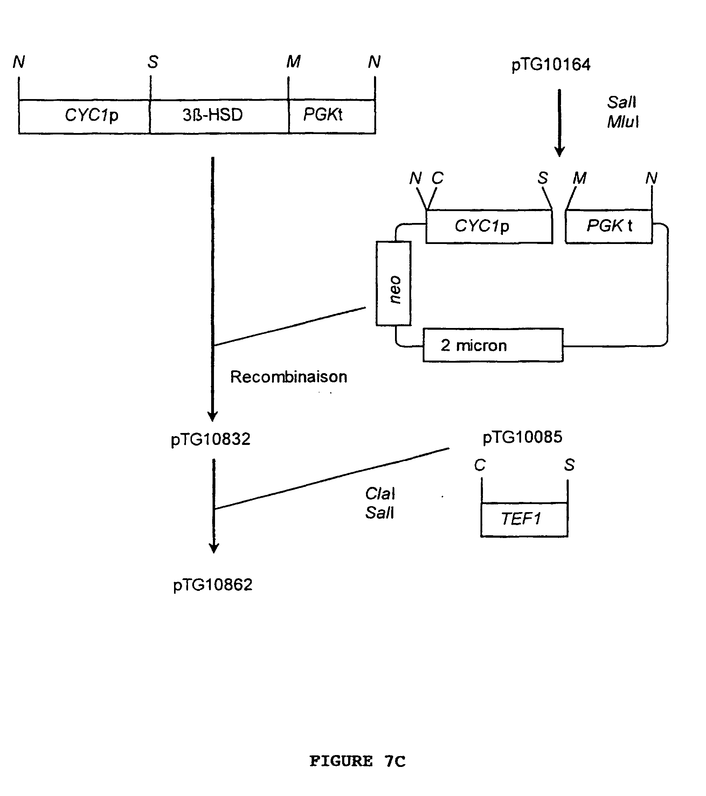

Les figures 7A, 7B et 7C représentent le schéma de construction des plasmides d'expression de la 3β-HSD humaine chez la levure, pTG10832 et pTG10862. La figure 7A décrit l'obtention du fragment *Msc*I-*Mlu*I contenant la séquence codante pour la 3β-HSD humaine. La figure 7B décrit l'obtention du fragment *Not*I contenant le bloc d'expression CYClₚ/3β-HSD/PGKₜ. La figure 7C décrit l'obtention du plasmide pTG10832 et du plasmide pTG10862.

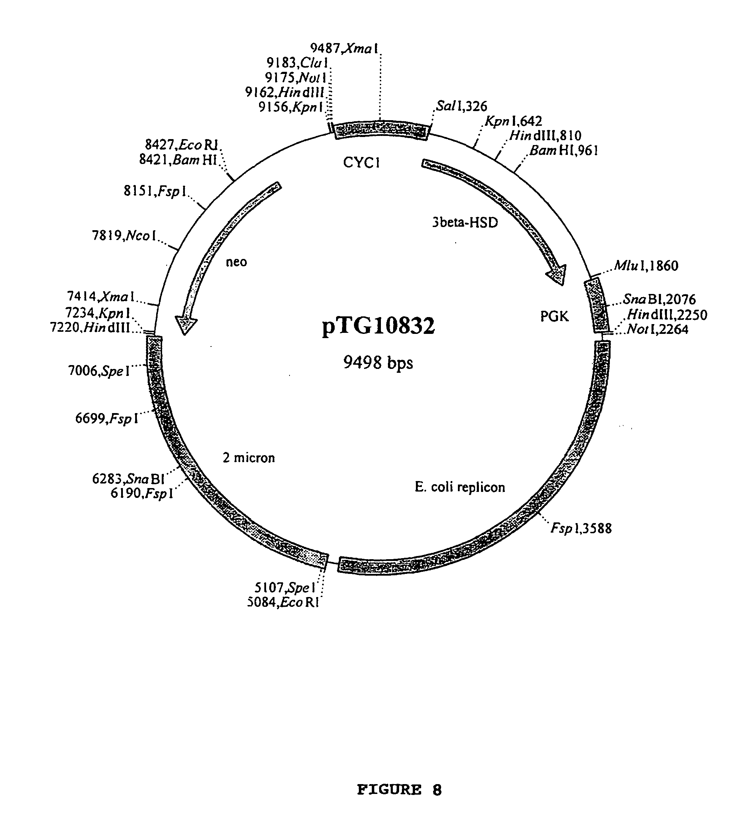

La figure 8 représente une carte de restriction du plasmide pTG10832.

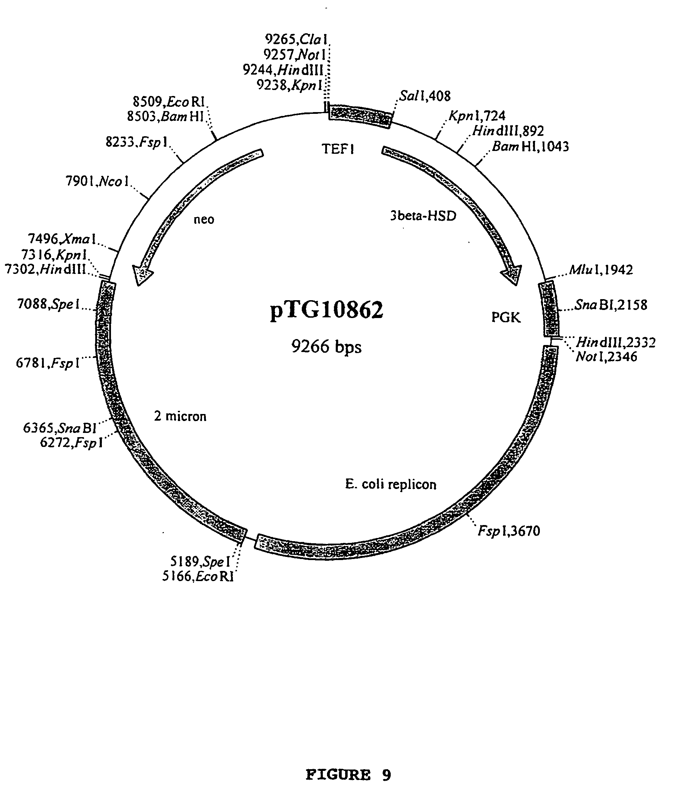

La figure 9 représente une carte de restriction du plasmide pTG10862.

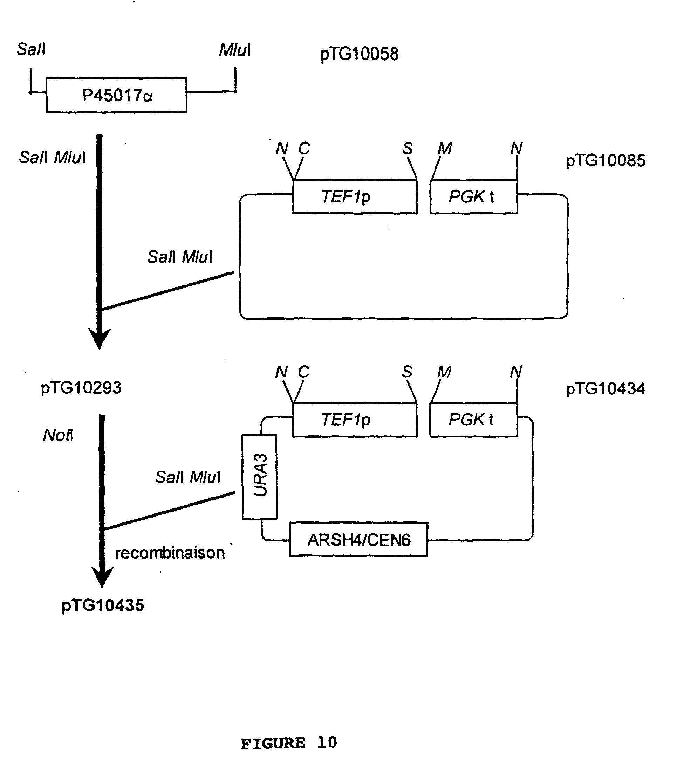

La figure 10 représente le schéma de construction du plasmide d'expression pTG10435.

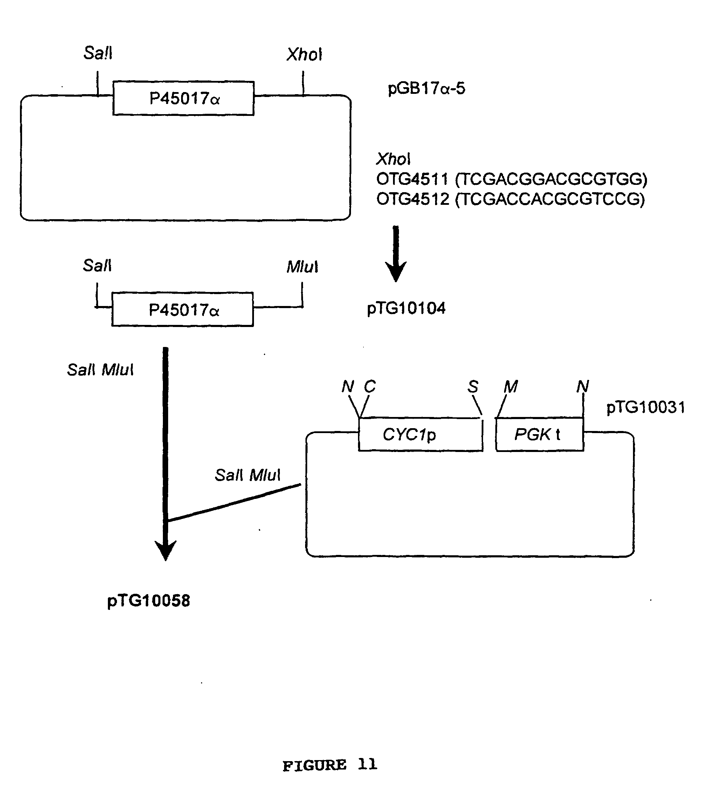

La figure 11 représente le schéma de construction du plasmide pTG10058.

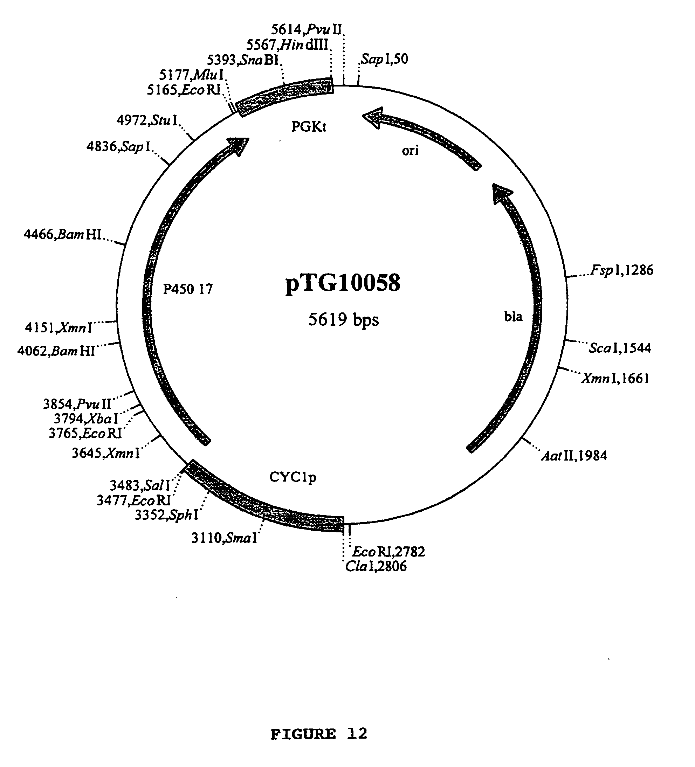

La figure 12 représente une carte de restriction du plasmide pTG10058.

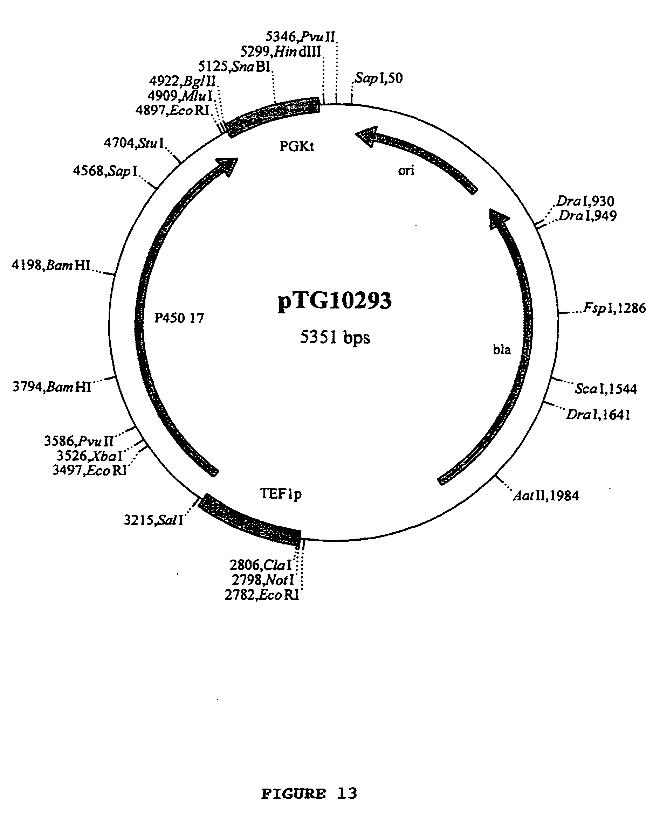

La figure 13 représente une carte de restriction du plasmide pTG10293.

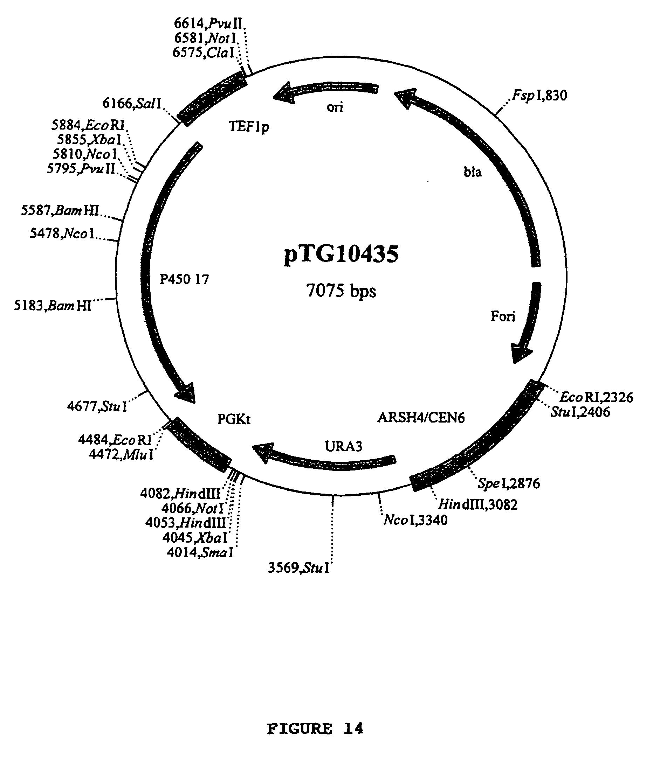

La figure 14 représente une carte de restriction du plasmide pTG10435.

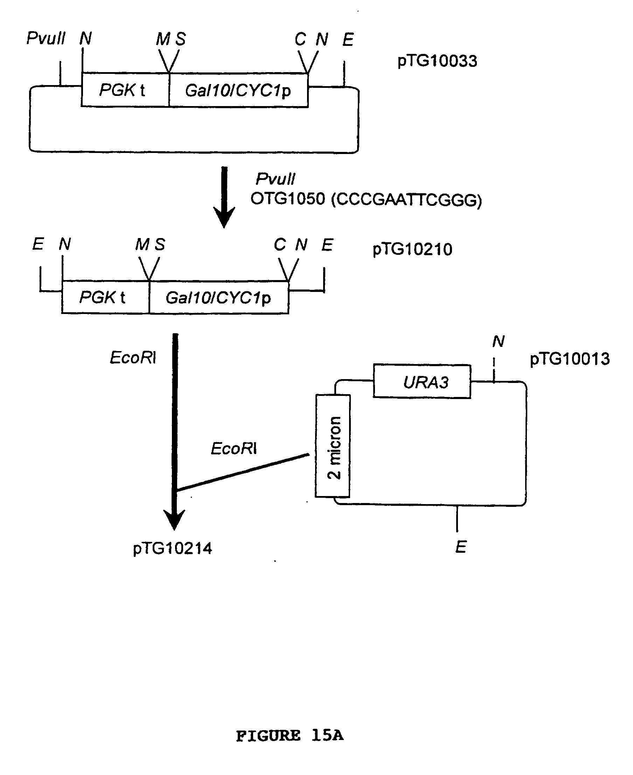

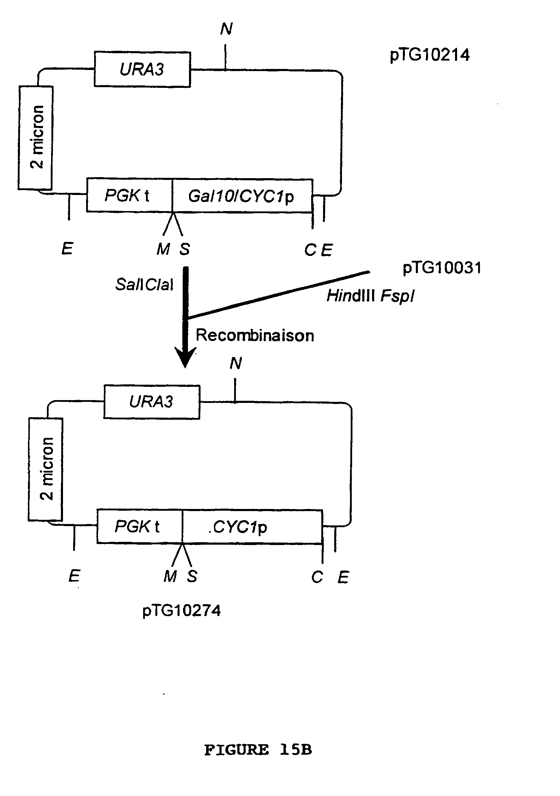

Les figures 15A et 15B représentent le schéma de construction du plasmide pTG10274 :

La figure 15A décrit l'obtention du plasmide pTG10214. La figure 15B décrit l'obtention du plasmide pTG10274.

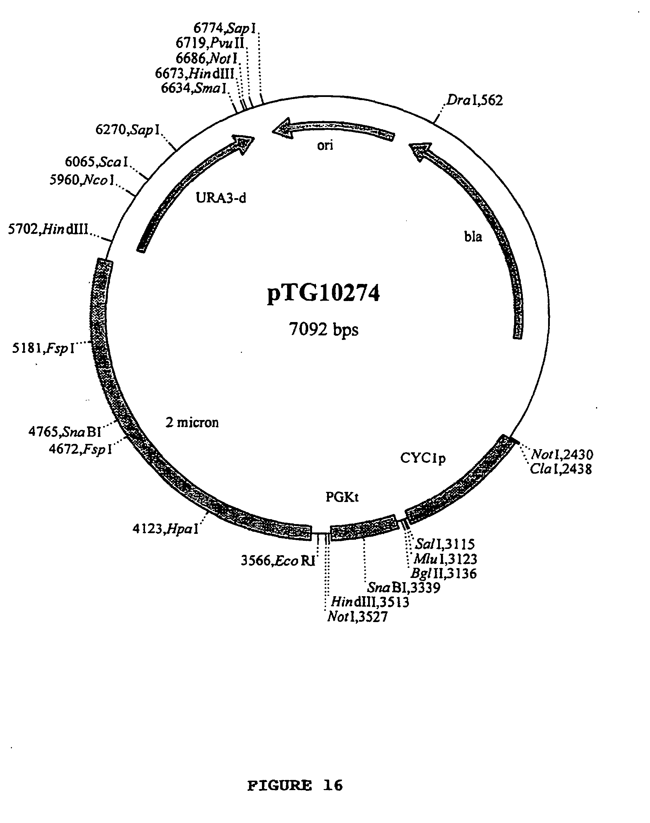

La figure 16 représente une carte de restriction du plasmide pTG10274.

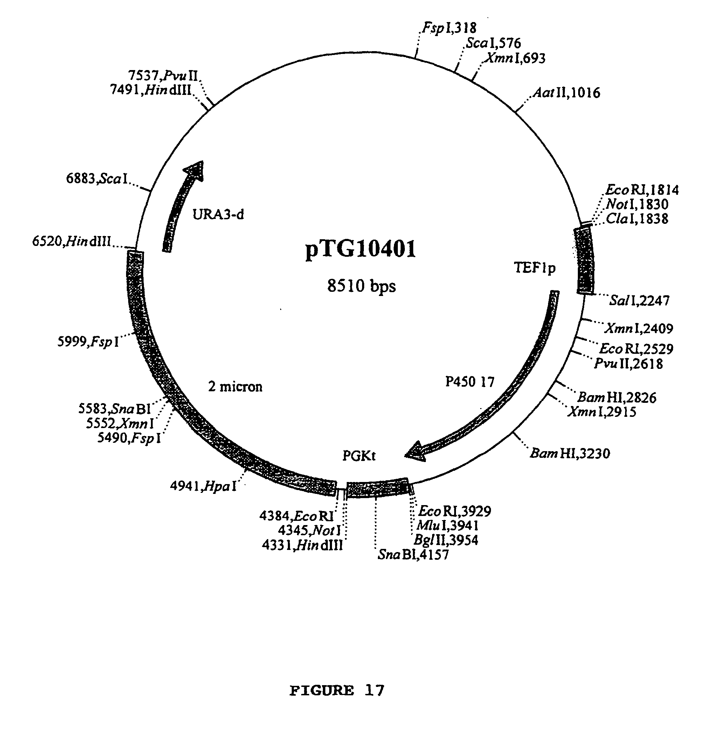

La figure 17 représente une carte de restriction du plasmide pTG10401.

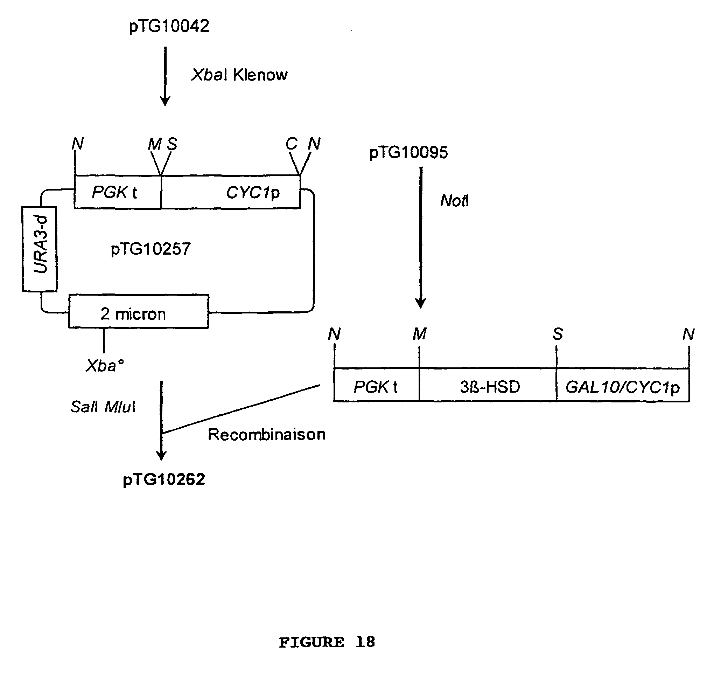

La figure 18 représente le schéma de construction du vecteur d'expression pTG10262.

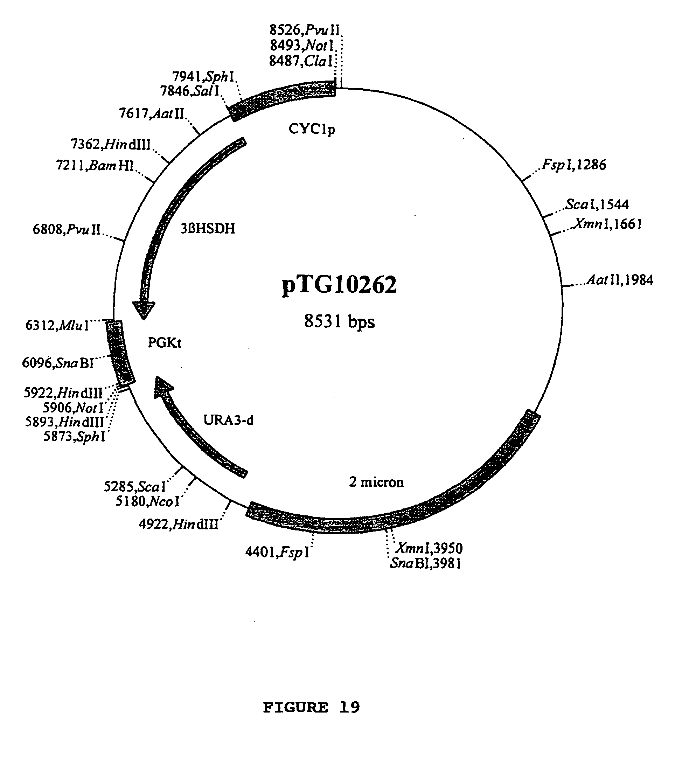

La figure 19 représente une carte de restriction du plasmide pTG10262.

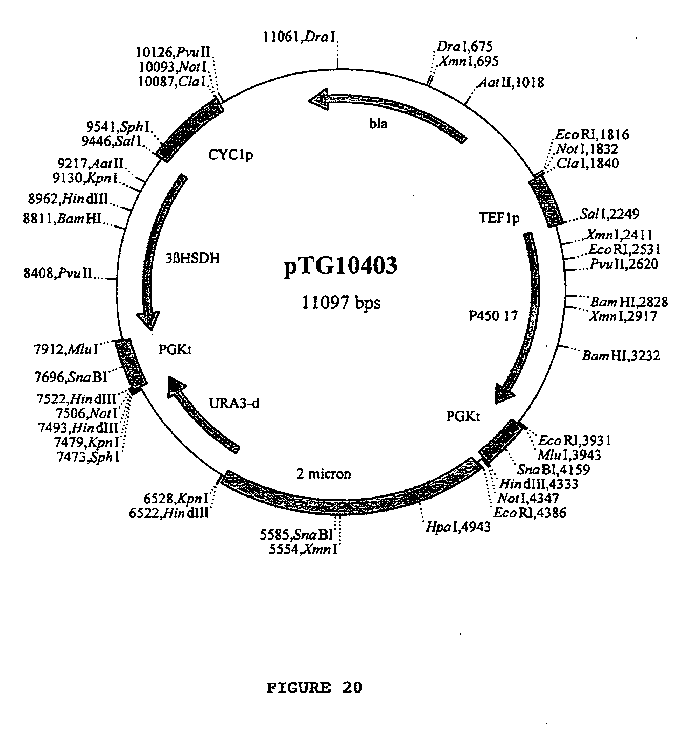

La figure 20 représente une carte de restriction du plasmide pTG10403.

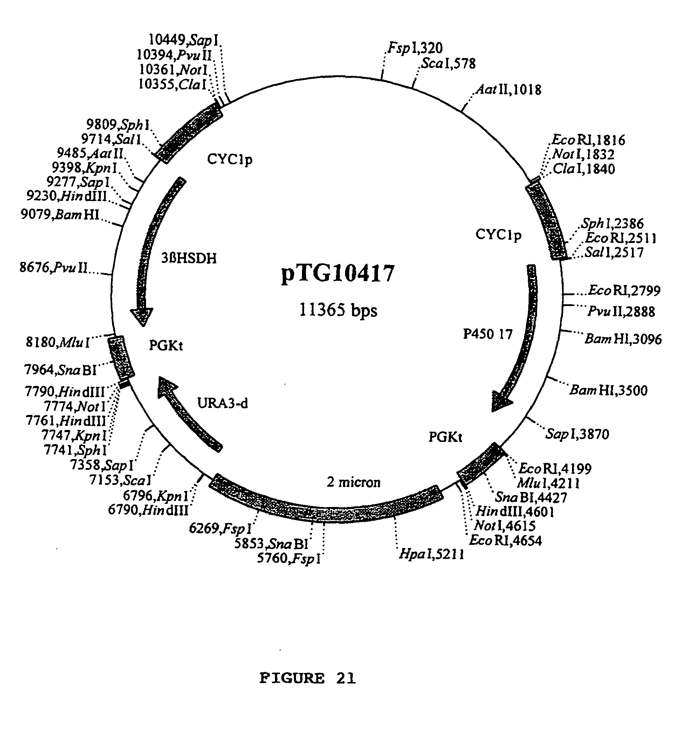

La figure 21 représente une carte de restriction du plasmide pTG10417.
(Abréviations des enzymes de restriction : *S, Sal*I *; N,Not*I ; *B*II, *Bgl*II *M*, *Mlu*I*, C, Cla*I *; N°,* site *Nco*I perdu ; XbaI°, site *Xba*I perdu ; *E,Eco*RI).

### EXEMPLE 1 : Identification de l'activité APAT de la levure.

### A - Acétylation in vivo de la prégnénolone par la levure.

La souche TGY73.4 a été cultivée à 28°C dans 10 ml de milieu YPD (Difco) inoculé à A600=0,1 à partir d'une préculture de 24 h et dans lesquels ont été ajoutés 100 µl d'une solution de prégnénolone à 10 mg/ml dans un mélange tergitol (Sigma)/ éthanol (1:1). Les stéroïdes formés ont été identifiés sur des aliquots de 250 µl de bouillon prélevés à intervalles de temps pendant 10 h. Après extraction avec 2 ml de dichlorométhane, les phases organiques ont été évaporées sous azote, puis les résidus obtenus ont été redissous dans de l'acétonitrile. Les stéroïdes ont été analysés par RP-HPLC sur une colonne Ultrasphère ODS (Beckman) avec successivement comme éluant : acétonitrile à 60 % dans l'eau pendant 10 mn, puis acétonitrile variant de 60 à 80 % dans l'eau pendant 5 mn, puis acétonitrile à 80 % pendant 5 mn au débit de 1 ml/mn, à 45°C et avec une détection à 205 nm.

Les chromatogrammes obtenus (figure 2B) montrent que la prégnénolone est métabolisée en un produit plus apolaire et ayant un TR identique à celui de la prégnénolone acétate standard. La figure 2A montre que la prégnénolone est rapidement convertie par la levure en son métabolite.

Après traitement alcalin (KOH à 6 % dans du méthanol), le métabolite observé libère un produit ayant un TR identique à celui de la prégnénolone. L'identification du métabolite à l'acétate de prégnénolone a été ensuite confirmée par spectrométrie de masse.

### B - Purification de l'enzyme ayant l'activité APAT.

La souche TGY73.4 a été cultivée en fermenteur de 10 litres dans le milieu Kâppeli (Fiechter et al., 1981) enrichi en glucose à 160 g/l à 30°C jusqu'à A600=30. Les cellules ont été séparées par centrifugation, lavées à l'eau puis remises en suspension dans 4 litres de tampon Tris-HCl 20 mM, pH 8,0 à 4°C (tampon A) contenant 1 mM de PMSF. Les cellules ont été soumises à un éclatement dans un homogénéiseur Manton Gaulin sous une pression de 1000 psi. Le lysat cellulaire obtenu a été centrifugé à 12.000 xg pendant 15 mn à 4°C, puis du chlorure de zinc a été ajouté au surnageant à une concentration finale 40 mM. Le pH a été ajusté à 5.5 avec HCl 1N et la précipitation a été réalisée pendant 30 mn à 4°C. Après centrifugation à 10.000 xg pendant 10 mn à 4°C, le précipité isolé a été remis en suspension dans 3 litres de tampon A contenant de l'EDTA 100 mM et du PSFM 1 mM. Après élimination de l'EDTA par diafiltration sur une cartouche Y10S10 (Amicon) contre 30 litres du tampon A, le rétentat a été chargé à la vitesse de 35 ml/mn et à 4°C sur une colonne de 1,5 litres de DEAE-Sephacel (Pharmacia) préalablement équilibrée avec le tampon A. Après lavage de la colonne avec le tampon A, puis avec le tampon A contenant du NaCl 0,15M, l'activité APAT a été éluée avec le tampon A contenant du NaCl 0,4M. Les fractions de DEAE-Sephacel contenant l'activité APAT, mesurée comme indiqué ci-dessus dans "Matériels et Méthodes générales", ont été réunies, additionnées de NaCl à la concentration finale de 2M, puis chargées à la vitesse de 15 ml/mn et à 4°C sur une colonne de 500 ml de Phényl-Sepharose (Pharmacia) préalablement équilibrée dans le tampon A contenant du NaCl 2M. Après lavage de la colonne avec le tampon A contenant NaCl 0,5M, l'activité APAT est éluée avec 1,5 litres d'un gradient linéaire de cholate de sodium variant de 0 à 1 % dans le tampon A. Les fractions contenant l'activité APAT ont été réunies, puis concentrées par ultrafiltration sur une membrane YM10 (Amicon), puis conservées à ∼80°C jusqu'à utilisation.

L'ensemble du procédé a été répété une fois de façon à préparer du matériel en quantité suffisante pour continuer la purification.

Le matériel purifié à partir des deux préparations ci-dessus a été décongelé, puis chargé à la vitesse de 4 ml/mn et à 4°C sur une colonne de 100 ml de Q-Sepharose Fast Flow (Pharmacia) préalablement équilibrée dans le tampon A. Après lavage de la colonne avec le même tampon, l'activité APAT a été éluée avec 500 ml d'un gradient linéaire de NaCl variant de 0 à 1M dans le même tampon. Les fractions de Q-Sepharose contenant l'activité APAT ont été réunies puis chargées directement à la vitesse de 2,5 ml/mn et à 4°C sur une colonne de 7 ml de cholate de sodium immobilisé sur des billes de Sepharose (Pharmacia) équilibrée préalablement avec du tampon A contenant du NaCl 0,5M. Après lavage de la colonne avec le même tampon, l'activité APAT a été éluée avec 100 ml d'un gradient linéaire de cholate de sodium variant de 0 à 1 % dans le même tampon. Les fractions contenant l'activité APAT ont été réunies, concentrées par ultrafiltration sur une membrane YM10 (Amicon) jusqu'à une concentration en protéine de 1,8 mg/ml, puis conservées à -80°C.

L'activité APAT a été ainsi purifiée environ 500 fois sur la base de l'activité spécifique et avec un rendement d'environ 16 % comme montré au tableau 1 suivant :

**Tableau 1**

| Stade | Activité APAT (unités) | Protéine (mg) | Activité spécifique (unités/mg) | Purification | Rendement |
|---|---|---|---|---|---|
| Surnageant 12.000g | 8 057 | 42 242 | 0,19 | 1 | 100 |
| Précipitation au zinc | 10 453 | 28 040 | 0,37 | 1,9 | 129 |
| DEAE-Sephacel | 6 399 | 5 009 | 1,28 | 6,7 | 79 |
| Phényl-Sepharose | 3 316 | 489 | 6,78 | 35,7 | 41 |
| Q-Sepharose | 1 712 | 39 | 43,9 | 231 | 21 |
| Cholate-Sepharose | 1 300 | 13 | 100 | 526 | 16 |

La moitié du matériel semi-purifié obtenu ci-dessus (environ 6 mg de protéine) a été ensuite décongelée puis additionnée de PEG 4000 (Prolabo) à la concentration finale de 20 % (p/v) pour éliminer le cholate et le NaCl. Après agitation pendant 30 mn à 4°C, le précipité a été recueilli par centrifugation à 12.000 xg pendant 30 mn à 4°C, puis redissous dans 4 ml de tampon A. La solution ainsi obtenue a ensuite été chargée à la vitesse de 1 ml/mn sur une colonne de MonoP HR5/20 (Pharmacia) préalablement équilibrée avec le tampon bis-Tris 25 mM pH 6,3. L'activité APAT a été éluée avec le tampon Polybuffer 74 pH 4,0 (Pharmacia). Des fractions de 1 ml ont été recueillies en présence chacune de 50 µl de tampon Tris-HCl 2M, pH 8.0 de façon à limiter l'inactivation de l'enzyme à pH acide. Les fractions 14, 15 et 16 (figure 3(A)) contenant l'activité APAT la plus élevée, mesurée comme indiquée ci-dessus, ont été réunies, concentrées par ultrafiltration sur membrane YM10, puis conservées à -80°C avant utilisation. La fraction active ainsi obtenue a été soumise à une SDS-PAGE sur gel à 10 % de polyacrylamide. Plusieurs bandes ont été révélées par coloration au bleu de Coomassie avec une bande majoritaire d'un PM apparent de 62kDa (figure 3(B)), identique au PM déterminé par filtration sur gel de Superose 6 (Pharmacia) et correspondant à l'activité APAT.

### C - Propriétés de l'APAT.

### a) Spécificité de substrat

Selon la méthode indiquée ci-dessus pour l'acétyl CoA et la prégnénolone, en utilisant différents donneurs d'acyle ou différents substrats stéroïdes, l'APAT semi-purifiée transfert l'acétate sur des 3β-ol, delta⁴ ou delta⁵-stéroïdes avec une efficacité comparable alors que le transfert est faible sur les estrogènes et non détectable sur les stérols et avec une préférence marquée pour l'acétyl-CoA comme donneur d'acyle.

Le tableau 2 suivant, dans lequel pour a) les essais sont effectués avec 30 µM de chaque stéroïde testé et 100 µM de [³H]acétyl-CoA et pour b) les essais sont effectués avec 100 µM de chaque donneur d'acyle et 30 µM de [³H] prégnénolone, montre les résultats obtenus :

**Tableau 2**

| Substrat a) Activité relative (%) | | Substrat b) Activité relative (%) | |
|---|---|---|---|
| Prégnénolone | 100 | acétyl-CoA | 100 |
| 17α-OH prégnénolone | 89 | propionyl-CoA | 33 |
| DHEA | 104 | butyryl-CoA | 16 |
| 4-pregnène-3βol-20-one | 70 | hexanoyl-CoA | 18 |
| 5β-pregnane-3βol-20-one | 1,8 | oléoyl-CoA | non détectée |
| 17β-oestradiol | 5 | | |
| oestrone | 2,5 | | |
| cholestérol | 0,6 | | |
| ergostérol | 0,4 | | |

### b) Inhibition

L'activité APAT est fortement inhibée par les réactifs des groupements sulfhydryles tels que NEM et DTNB. L'inhibition est complète en présence de chlorure de zinc (1 mM).

### D - Séquence partielle en acides aminés.

La séquence partielle en acides aminés a été déterminée après digestion à la trypsine sur des coupes de gel selon la méthode décrite par Rosenfeld et al. (1992).

A partir des deux tiers du concentrat obtenu ci-dessus puis séparé par SDS-PAGE, la bande 62kDa a été découpée puis incubée avec de la trypsine (Promega). Les peptides générés ont été ensuite séparés par RP-HPLC sur une colonne Vydac 218TP (1,5 x 125 mm) à la vitesse de 100 µl/mn, en utilisant un gradient linéaire d'acétonitrile variant de 0 à 60 % en 80 mn, puis de 60 à 100 % en 20 mn dans une solution de TFA à 0,1 % dans l'eau puis ont été soumis à un séquençage en acides aminés.

La séquence N-terminale a été déterminée sur un séquenceur de protéine Model 477A couplé à un analyseur HPLC de PTH-amino acids (Applied Biosystems).

Parmi les échantillons séquencés, deux pics x et y donnent une séquence non ambiguë constituée respectivement des 10 et 16 acides aminés suivants :
pour le pic x : ISEQFKKDDF
pour le pic y : LIELISPVIIPLGNPK

Les deux séquences peptidiques ainsi obtenues ont été utilisées pour cribler la base de données du génome de *S. cerevisiae.* Une protéine de 62kDa dont la séquence contient exactement les séquences des deux peptides ci-dessus a pu être identifiée (Mips, accession S64491, Hebling et al., May 1996). Cette protéine dont la séquence est montrée à la figure 4 et dont la fonction n'a pas été décrite serait codée par un gène au locus *YGR177c.* Sur la base d'une identité d'environ 37 % de séquence en acides aminés entre la protéine de l'invention ayant une activité acyltransférase et le produit du gène *ATF1* chez *S. cerevisiae* décrit par Fujii et al. (1994) et auquel a été attribué une activité alcool acétyltransférase, nous utiliserons la dénomination *ATF2* pour le gène codant pour la protéine responsable de l'activité APAT chez *S. cerevisiae.*

### EXEMPLE 2 : Construction de souches de levure ayant le gène ATF2 interrompu par le gène URA3 et ayant perdu l'activité APAT (atf2-Δ::URA3).

### A) Ciblage du gène ATF2.

Le gène *URA3* de *S. cerevisiae* a été introduit par substitution d'une partie choisie du gène *ATF2* de *S. cerevisiae* permettant une sélection ultérieure des souches mutantes par prototrophie à l'uracile.

Le marqueur de sélection *URA3* a été associé au gène *ATF2* par double fusion PCR selon la méthode décrite par Amberg et al., 1995. La stratégie suivie montrée à la figure 5 comprend au total 4 réactions de PCR. Les deux premières réactions (noté PCR1) permettent respectivement d'amplifier les régions 5' et 3' flanquant le site d'insertion du marqueur *URA3* dans le gène cible *ATF2* interrompu et la troisième réaction (noté PCR2) permet d'amplifier le gène marqueur *URA3*. La double fusion (noté PCR3) permet enfin l'association des régions 5' et 3' du gène cible *ATF2* au gène marqueur *URA3* (noté 5'*ATF2-URA3-3' ATF2*).

Dans un premier temps, un échantillon de cellules entières de la souche Fy1679 utilisée comme source d'ADN du gène cible *ATF2* a été amplifié dans le tampon PCR contenant du dNTP 2 mM (Pharmacia) dans les conditions suivantes : 25 cycles ; 93°C, 30 sec ; 54°C, 2 mn ; 68°C, 3 mn suivi d'une élongation de 5 mn à 72°C ; polymérase Ampli Taq (Perkin Elmer) .

D'une part, la région 5' du gène *ATF2* a été amplifiée par PCR en utilisant comme amorces directe et indirecte les oligonucléotides ayant les séquences suivantes : et qui contiennent une région homologue à la région 5' de la séquence du gène *ATF2* (SGD : *YGR177c*) et en ajoutant un site de restriction *Sal*I pour l'OTG10841.

D'autre part, la région 3' du gène ATF2 a été amplifiée par PCR en utilisant comme amorces directe et indirecte les oligonucléotides ayant les séquences suivantes : et qui contiennent des régions homologues à la région 3' de la séquence du gène *ATF2* (SGD : *YGR177*c) et en ajoutant un site de restriction MluI pour l'OTG10842.

Dans un deuxième temps, le gène *URA3* de *S. cerevisiae* a été amplifié par PCR en utilisant comme amorce directe l'oligonucléotide ayant la séquence : qui contient une séquence homologue à la région 5' de la séquence du gène URA3 publiée (Rose et al., 1984 ; GenBank : YSCODCD accession : K02207 ; SGD : YEL021w) associée à une séquence homologue à la région 5' du gène ATF2 (complémentaire à l'OTG10844) et comme amorce indirecte l'oligonucléotide ayant la séquence : qui contient une séquence homologue à la région 3' du gène *URA3* associée à une séquence homologue à la région 3' du gène *ATF2* (complémentaire à l'OTG10846). Un échantillon de 20 ng d'ADN du gène *URA3,* isolé à partir du vecteur navette *E. coli*-levure pTG10021 (Degryse et al., 1995) par digestion avec l'enzyme de restriction *Hind*III a été amplifié dans les conditions indiquées ci-dessus.

Les produits de PCR respectivement obtenus ont été purifiés en utilisant la trousse "Geneclean kit" (Bio 101 Inc, La Jolla, USA), puis soumis à la réaction de double fusion en utilisant comme amorces les oligonucléotides OTG10841 et OTG10842 ci-dessus dans les conditions d'amplification utilisées pour les réactions préalables de PCR avec un programme de 20 cycles.

Après purification du produit final de fusion qui contient les régions flanquantes du gène ATF2 fusionnées au gène *URA3* fonctionnel, la présence du gène *URA3* a été confirmée par digestion avec l'enzyme de restriction EcoRV qui montre la présence de ce site dans le matériel amplifié.

### B) Génération de souches de levure atf2-Δ::URA3.

Le produit de fusion obtenu ci-dessus a été transformé directement dans les cellules compétentes de la souche Fy1679 ou de la souche TGY73.4 et les transformants ont été sélectionnés par croissance sur le milieu SD (F. Sherman, 1991) en présence des exigences nutritionnelles de la souche et en absence d'uracile.

A partir de clones isolés, la nouvelle association entre la région 5' du gène *ATF2* et le gène *URA3* (5'*ATF2-URA3-3'ATF2*) a été montrée par amplification par PCR sur des cellules entières en utilisant les amorces OTG10841 et OTG10845 ci-dessus. L'absence de l'activité APAT a été ensuite montrée in vitro selon le test décrit à partir d'homogénat celullaire, comparativement à la souche parente qui montre une activité APAT marquée.

Les souches répondant à ces critères ont ainsi été caractérisées comme des souches mutantes *atf2,* noté *atf2-Δ::URA3*. Une souche mutante ainsi obtenue à partir de la souche parente Fy1679 a été dénommée TGY156 et une souche mutante obtenu à partir de la souche parente TGY73.4 a été dénommée TGY158.

Un échantillon de la souche TGY156 a été déposé à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1977.

Un échantillon de la souche TGY158 a été déposé à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1976.

### C) Stabilisation de la prégnénolone in vivo dans les cultures de souches de levure atf2-Δ::URA3.

Les cellules de la souche TGY158 obtenue ci-dessus ont été inoculées à A600=0,1 dans le milieu YPD (Difco) contenant 100 µg/ml de prégnénolone. Après 16 heures d'incubation à 28°C, les stéroïdes ont été extraits avec du dichlorométhane et analysés par RP-HPLC comme indiqué ci-dessus. La figure 6(B) montre que le mutant TGY158 a perdu la capacité d'estérifier la prégnénolone alors que dans les mêmes conditions de culture, la souche parente TGY73.4 convertit la prégnénolone en prégnénolone acétate (figure 6(A)).

Ces résultats permettent de conclure que le produit du gène *ATF2* est responsable de l'estérification de la prégnénolone par la levure alors que l'interruption du gène *ATF2* n'a pas conduit à des modifications apparentes de la croissance cellulaire dans des conditions normales.

### EXEMPLE 3 : Construction d'une souche de levure ayant le gène ATF2 interrompu par le gène URA3 et exprimant la 3β-HSD

Des plasmides 2µ portant une séquence d'ADNc codant pour la 3β-HSD humaine sous le contrôle du promoteur *CYC1* de *S. cerevisae* ou du promoteur *TEF1* de *S. cerevisae* et portant le gène de résistance à G418 ont été construits selon le schéma des figures 7A à 7C, puis transformés dans des souches de levure mutantes *atf2-Δ::URA3.*

Dans un premier temps, un vecteur de transfert pTG10095, contenant la séquence d'ADNc codant pour la 3β-HSD humaine type II décrit par E. Rhéaume et al., 1991 flanquée par les sites *Sal*I et *Mlu*I et située en aval du promoteur de levure *GAL10*/*CYC1,* a été généré de la façon suivante :
La séquence codante pour la 3β-HSD a été sousclonée par E. Rhéaume et al., 1991 comme un fragment de restriction *Sal*I*-Not*I dans les mêmes sites du vecteur Bluescript II (Stratagène). Le vecteur obtenu décrit par E. Rhéaume et al., 1991 contient un site *Not*I localisé à l'extrémité 3' de la séquence codante pour la 3β-HSD. Ce vecteur a été ensuite digéré par l'enzyme de restriction *Not*I, puis traité par le fragment de Klenow en présence de dNTP afin de remplir les extrémités cohésives, puis religaturé en présence de l'oligonucléotide ayant la séquence suivante : préalablement phosphorylé et hybridé sur lui-même, de façon à introduire un site *Mlu*I. Le vecteur pTG10082 (fig. 7A) ainsi obtenu contient la séquence codante pour la 3β-HSD contenant un site *Bgl*II et bordée par les sites *Sal*I et *Mlu*I tandis que le site *Not*I a été perdu. Ce vecteur contient encore la région naturelle 5' non codante identifiée par la présence d'un site *Bgl*II.

Afin de rapprocher le site Sa*l*I de l'ATG initiateur en amont duquel on désire introduire le promoteur *GAL10*/*CYC1,* le vecteur pTG10082 a été digéré par l'enzyme de restriction *Msc*I dont le site est situé dans la région 5' non codante et juste en amont de l'ATG initiateur et par l'enzyme de restriction *Mlu*I. Le fragment *Msc*I-*Mlu*I de 1,8 kb, contenant la séquence codante pour la 3β-HSD (fig. 7A), a été isolé puis ligaturé dans le plasmide pTG10033 (E. Degryse et al., 1995) contenant le promoteur *GAL10*/*CYC1*, préalablement digéré par l'enzyme de restriction *Sal*I*,* puis traité par le fragment de Klenow en présence de dNTP, puis digéré par l'enzyme de restriction *Mlu*I. Le vecteur pTG10095 (fig. 7B) est ainsi obtenu.

Dans un second temps, le vecteur de recombinaison pTG10268 contenant le plasmide 2µ de levure, un replicon *d'E. coli,* une cassette d'expression *CYCl*_{*prom*}*-PGK*_{*term*} et le marqueur de sélection *LEU2* (fig. 7B), a été construit. Ce vecteur est identique au vecteur pTG10159 décrit (E. Degryse et al., 1995), à l'exception du site XbaI contenu dans la région 2µ qui a été remplacé par un marqueur *Xba*I° obtenu par remplissage du site naturel *Xba*I en présence du fragment de Klenow, puis par religature.

Le plasmide d'expression pTG10268 a été ensuite généré par recombinaison homologue par introduction du bloc d'expression contenant le promoteur *GAL10*/*CYC1p,* obtenu à partir du plasmide pTG10095 préparé ci-dessus puis digéré par l'enzyme de restriction *Not*I, dans le plasmide pTG10260 préalablement digéré par les enzymes de restriction *Sal*I et *Mlu*I. Le plasmide pTG10268 (figure 7B) contient la séquence codante pour la 3β-HSD humaine type II sous le contrôle du promoteur *CYC1.*

Le plasmide d'expression pTG10862 contenant la séquence codante pour la 3β-HSD sous le contrôle du promoteur *TEF1* a été ensuite construit de la façon suivante (figure 7C) :

Le plasmide pTG10832 (figure 8) a d'abord été construit par recombinaison homologue entre le fragment *Not*I obtenu à partir du plasmide pTG10268 préparé ci-dessus, puis digéré par l'enzyme de restriction *Not*I et le plasmide de recombinaison pTG10164 (E. Degryse et al., 1995) préalablement digéré par les enzymes de restriction *Sal*I et *Mlu*I.

Le plasmide d'expression pTG10862 a été ensuite obtenu par introduction du promoteur *TEF1,* contenu dans le fragment *Cla*I-*Sal*I isolé à partir du plasmide pTG10085 (E. Degryse et al., 1995), à la place du promoteur *CYC1* excisé par digestion du plasmide pTG10832 construit ci-dessus par les enzymes de restriction *Cla*I et *Sal*I.

Le plasmide pTG10862 ainsi obtenu (figure 9) et contenant la séquence d'ADNc codant pour la 3β-HSD humaine type II a été transformé respectivement dans la souche parente TGY73.4 ou dans son mutant *atf2-*Δ*::URA3* correspondant à la souche TGY158 obtenue à l'exemple 2 ainsi que dans la souche parente FY1679 ou dans son mutant *atf2-Δ::URA3* correspondant à la souche TGY156 obtenue à l'exemple 2. Les transformants ont été isolés comme indiqué ci-dessus sur le milieu YPD (Difco) contenant du G418 à 250 µg/ml.

Les colonies-candidates ainsi obtenues ont été ensuite précultivées sur le milieu SD contenant les éléments nutritionnels nécessaires pour chaque souche (histidine et uracile pour la souche TGY73.4 ; histidine pour TGY158 ; tryptophane, histidine, leucine et uracile pour la souche FY1679 ; tryptophane, histidine et leucine pour la souche TGY156 ; à la concentration de 100 µg/ml chacun), puis inoculées dans un milieu contenant de 100 µg/ml de prégnénolone. Après 24 h de croissance et bioconversion à 28°C, les stéroides ont été extraits et mesurés par RP-HPLC comme indiqué ci-dessus. Les résultats obtenus, mesurés sur 3 clones de chaque souche, sont montrés dans le tableau 3 suivant :

**Tableau 3**

| **Souche** | **Plasmide** | **Prégnénolone (µg/ml)** | **Progestérone (µg/ml)** |
|---|---|---|---|
| TGY73.4 | pTG10862 | 1 | 0 |
| TGY158 | " | 94 | 15,2 |
| FY1679 | " | 1 | 1,3 |
| TGY156 | " | 100 | 13 |

Ces résultats montrent que le substrat prégnénolone est récupéré presque quantitativement dans les souches mutantes TGY156 ou TGY158 dans lesquelles un début de bioconversion de la prégnénolone en progestérone est observée alors que la disparition de la prégnénolone est complète dans les souches parentes TGY73.4 ou FY1679 qui ne produisent pas ou très peu de la progestérone, mais accumulent de l'acétate de prégnénolone comme cela a été montré à l'exemple 2.

Un échantillon de la souche transformée TGY158/pTG10862 a été déposé à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1978.

### Exemple 4 : Construction d'une souche de levure ayant le gène ATF2 interrompu par TEF1ₚᵣₒₘ/PGKₜₑᵣₘ (atf2-Δ:: TEF1/PGK).

La souche TGY186, qui est une souche dérivée de la souche TGY156 (*atf2- Δ::URA3)* décrite à l'exemple 2 dans laquelle le gène *URA3* au locus *ATF2* a été remplacé par le bloc d'expression *TEF1*_{*prom*}/*PGK*_{*term*}*,* a été construite de la façon suivante :

Dans un premier temps, le bloc d'expression *TEF1*_{*prom*}/*PGK*_{*term*} a été associé au gène *ATF2* par double fusion PCR en suivant les conditions décrites à l'exemple 2, mais en utilisant le bloc d'expression *TEF1*_{*prom*}/*PGK*_{*term*} de *S. cerevisiae* décrit par E. Degryse et al., 1995 au lieu du marqueur de sélection *URA3*. Les deux premières réactions de PCR (PCR1) permettant respectivement d'amplifier les régions codantes 5' et 3' du gène *ATF2* flanquant le site d'insertion du bloc *TEF1*_{*prom*}/*PGK*_{*term*} dans le gène cible *ATF2* interrompu, ont été réalisées en utilisant respectivement comme amorces directe et indirecte pour la région 5', les oligonucléotides ayant les séquences suivantes : et pour la région 3', les oligonucléotides ayant les séquences suivantes : et Les amorces OTG11049 et OTG11050 ont été désignées pour introduire respectivement les sites de restriction *Sal*I et *Mlu*I.

La troisième réaction de PCR (PCR2) permettant d'amplifier le bloc *TEF1*_{*prom*}/*PGK*_{*term*} a été réalisée en utilisant respectivement comme amorces directe et indirecte les oligonucléotides ayant les séquences suivantes : et qui introduisent les sites de restriction *Cla*I et *Hind*III. Finalement, l'association a été réalisée par double fusion des produits de PCR obtenus ci-dessus en utilisant comme amorces les oligonucléotides ayant les séquences OTG11049 (SEQ ID N°8) et OTG11050 (SEQ ID N°11) ci-dessus qui introduisent les sites de restriction *Sal*I et *Mlu*I aux sites de jonction *ATF2*.

Après purification, le produit final de fusion a ensuite été recombiné avec le gène *ATF2* contenu dans le plasmide pTG10885 construit comme indiqué ci-dessous et préalablement digéré avec les enzymes de restriction *Bst*I et *Stu*I. Le plasmide pTG10888, contenant le signal *TEF1*_{*prom*}/*PGK*_{*term*} aux sites *Cla*I et *Hind*III bordé par les régions flanquantes du gène *ATF2,* est ainsi obtenu.

La préparation du plasmide pTG10885 comprend l'amplification du gène *ATF2* à partir de la souche FY1679 en suivant les conditions décrites à l'exemple 2, mais en utilisant respectivement comme amorces directe et indirecte les oligonucléotides ayant les séquences OTG11049 (SEQ ID N°8) et OTG11050 (SEQ ID N°11) ci-dessus qui introduisent des sites de restriction *Sal*I et *Mlu*I. Dans le produit de PCR obtenu, ces sites ont été ensuite éliminés par digestion avec les enzymes de restriction *Sal*I et *Mlu*I, puis traitement avec le fragment de Klenow de la polymérase I *d'E. coli,* de façon à remplir les extrémités collantes. Le fragment obtenu a été ensuite ligaturé dans le vecteur d'expression pTG10031 décrit par E. Degryse et al., 1995, préalablement digéré avec les enzymes *Cl*aI et *Hind*III, puis traité avec le fragment de Klenow. Par transformation dans *E. coli,* le plasmide pTG10885 est ainsi obtenu, résultant de la ligation du site *Sal*I du produit de PCR, rempli en utilisant le fragment de Klenow de façon à obtenir la séquence GTCGA, avec le site *Hind*III du vecteur, rempli en utilisant le fragment de Klenow de façon à obtenir la séquence AGCTT, de manière à reconstruire le site *Hind*III (GTCGAAGCTT) (SEQ ID N°14) et à perdre le site *Cla*I. Le site *Cla*I du vecteur, rempli en utilisant le fragment de Klenow de façon a obtenir la séquence ATCG est perdu après ligation au produit de PCR. Le *signal TEF1*_{*prom*}/*PGK*_{*term*} a été ensuite excisé du plasmide pTG10888 sous forme d'un fragment *Not*I de 1,8 kb, puis a été échangé avec le marqueur *URA3* dans la souche TGY156 (*atf2-Δ*::*URA3*).

La souche TGY156, préparée à l'exemple 2, utilisée comme souche hôte a été co-transformée avec l'ADN excisé du plasmide pTG10888 et avec le vecteur de levure contenant une origine ARS, dénommé YRp7, décrit par Struhl et al., 1979 qui permet de complémenter l'exigence en tryptophane de la souche TGY156 et une détection sélective des colonies par leur résistance à l'acide 5-fluoroorotique (5-FO). 2 à 5 µg de l'ADN excisé du plasmide pTG10888 par digestion avec l'enzyme de restriction *Not*I et de plasmide YRp7 ont été introduits dans la souche TGY156 par la méthode à l'acétate de lithium (Ito et al., 1983). La sélection pour la complémentation de l'exigence en tryptophane de la souche a été ensuite réalisée après étalement sur des boîtes d'agar en milieu YNGB (Difco) enrichi en histidine et en leucine (100µg/ml de chacun). Les colonies candidates, prélevées à l'aide d'un cure-dent, ont été ensuite placées sur un milieu contenant du 5-FO préparé selon Boeke et al., 1984 puis la résistance au 5-FO a été confirmée sur le même milieu, la perte éventuelle du vecteur YRp7 dans les résistants à 5-FO étant indiquée par une exigence en tryptophane. Parmi les clones ainsi sélectionnés, l'association du gène *ATF2* avec *TEF1*_{*prom*} et *PGK*_{*term*} a été contrôlée par PCR. La souche TGY186 est ainsi obtenue.

### Exemple 5 : Construction d'une souche de levure ayant le gène ATF2 interrompu par TEF1ₚᵣₒₘ/PGKₜₑᵣₘ et exprimant P₄₅₀17α.

Un plasmide (pTG10435) contenant une origine de réplication de levure ARSH4/CEN6, le marqueur de sélection *URA3* et portant une séquence d'ADNc codant pour le cytochrome P₄₅₀17α bovin sous le contrôle du promoteur *TEF1* de *S. cerevisiae* a été construit selon le schéma de la figure 10, puis transformé dans une souche de levure mutante *atf2-Δ::TEF1*/*PGK* (TGY186) . Dans un premier temps, un plasmide pTG10058 contenant la séquence d'ADNc codant pour le cytochrome P₄₅₀17α. bovin décrit par Zuber et al., 1986, flanquée par les sites *Sal*I et *Mlu*I et située en aval du promoteur de levure *CYC1,* a été généré selon le schéma de la figure 11 : Le plasmide pGB17α-5 décrit dans la demande de brevet WO 89/10963 et contenant la séquence codante pour le cytochrome P₄₅₀17α bovin a été ouvert par digestion avec l'enzyme de restriction *Xho*I puis traité à la phosphatase alcaline. Après phosphorylation et hybridation, les oligonucléotides ayant les séquences suivantes : et ont été introduits dans le site *Xho*I générant le plasmide pTG10104. Le plasmide pTG10104 a été ensuite traité par les enzymes de restriction *Sal*I et *Mlu*I, puis introduit dans le plasmide pTG10031 décrit par E. Degryse et al., 1995 contenant le promoteur de levure *CYC1,* préalablement digéré avec les enzymes de restriction *Sal*I et *Mlu*I et traité avec la phosphatase alcaline. Le vecteur pTG10058 contenant l'ADNc codant pour le cytochrome P₄₅₀17α bovin est ainsi obtenu (figure 12). Le plasmide pTG10058 a été ensuite digéré par les enzymes de restriction *Sal*I et *Mlu*I et traité à la phosphatase alcaline. Le fragment *Sal*I-*MIu*I de 1,7kb contenant la séquence codante pour le cytochrome P₄₅₀17α bovin a été isolé, puis ligaturé dans le vecteur d'expression pTG10085 décrit par E. Degryse et al., 1995 et contenant le promoteur de levure *TEF1,* préalablement digéré par les enzymes *Sal*I et *Mlu*I. Le plasmide pTG10293 (figure 13) dans lequel la séquence codante pour le cytochrome P₄₅₀17α est sous le contrôle du promoteur *TEF1* est ainsi obtenu. Dans un second temps, le plasmide d'expression pTG10435 a été généré par recombinaison homologue entre le plasmide de recombinaison pTG10434 décrit par E. Degryse et al, 1995 et contenant la séquence ARSH4/CEN6, préalablement digéré par les enzymes *Sal*I et *Mlu*I et le fragment *Not*I de 2,8 kb obtenu à partir du plasmide pTG10293 préparé ci-dessus. Le plasmide pTG10435 ainsi obtenu (figure 14) et contenant la séquence codant pour le cytochrome P₄₅ₒ17α bovin sous le contrôle du promoteur *TEF1,* a été ensuite transformé respectivement dans la souche parente FY1679 ou dans la souche TGY186 (*atf2-Δ::TEF1*/*PGK*) préparée à l'exemple 4.

Les transformants ont été isolés comme indiqué ci-dessus sur le milieu YNBG (Difco) enrichi en tryptophane, histidine et leucine (100 *µ*g/ml de chacun). Les colonies ainsi obtenues ont été ensuite précultivées pendant 16 heures dans le milieu YNB (Difco) contenant 2 % de glucose et 0,5 % de casaminoacides, puis diluées dans du milieu frais à A600 = 0,2. Après 6 heures de croissance, 100 µ/ml de prégnénolone ou de progestérone ont été ajoutés. Après 48 heures de croissance et de bioconversion à 28°C, les stéroïdes ont été extraits et mesurés par RP-HPLC comme indiqué à l'exemple 1 en utilisant respectivement des standards de prégnénolone et de 17α-hydroxyprégnénolone ou de progestérone et de 17α-hydroxypro-gestérone.

Les résultats obtenus exprimés en *µ*g/ml sont montrés dans le tableau 4 suivant :

**Tableau 4**

| **Souche** | **FY1679/pTG10435** | **TGY186/pTG10435** |
|---|---|---|
| Substrat : Prégnénolone Prégnénolone | 0 | 67,1 |
| 17α-Hydroxyprégnénolone | 0 | 42,0 |
| Substrat : Progestérone Progestérone | 46,5 | 41,4 |
| 17α-Hydroxyprogestérone | 29,3 | 33,1 |

Ces résultats montrent que la capacité de bioconversion du cytochrome P₄₅₀17α exprimé à partir du vecteur pTG10435 est à peu près la même dans la souche sauvage (FY) ou dans son mutant *atf2* (TGY186) avec la progestérone comme substrat. D'autre part, avec la prégnénolone comme substrat, la même bioconversion est obtenue comparativement à la progestérone mais seulement avec le mutant *atf2* (TGY186). Dans la souche sauvage FY, à la fois le substrat et le produit sont acétylés et on ne détecte pas d'hydroxyprogestérone libre.

Un échantillon de la souche transformée TGY186/pTG10435 a été déposé à la Collection Nationale de Cultures de Microorganismes (CNCM) INSTITUT PASTEUR, 25, Rue du Docteur Roux 75724 PARIS CEDEX 15 FRANCE, le 20 janvier 1999 sous le numéro I-2119.

### Exemple 6 : Construction d'une souche de levure ayant le gène ATF2 interrompu par TEF1ₚᵣₒₘ/PGKₜₑᵣₘ et coexprimant la 3β-HSD et P₄₅₀17α.

Le plasmide pTG10417 (figure 21) contenant le réplicon de levure 2µ et deux blocs d'expression, l'un codant pour la 3β-HSD humaine, l'autre codant pour le cytochrome P₄₅₀17α bovin, tous les deux sous le contrôle du promoteur *CYC1* de *S. cerevisiae* et portant le marqueur de sélection *URA3*-d a été construit en suivant successivement les stades 1 à 3 décrits ci-dessous (figures 15A et B), puis les stades 4 à 6 décrits ci-dessous, puis transformé dans une souche de levure mutante *atf2-Δ::TEF1*_{*prom*}/*PGK*_{*term*} (TGY186).

### Stade 1 : construction du plasmide pTG10210

Le vecteur d'expression pTG10033 décrit par E. Degryse et al., 1995 et contenant le promoteur hybride de levure *GAL10*/*CYC1,* préalablement digéré par l'enzyme de restriction PvuII, a été traité par la phosphatase alcaline puis religaturé en présence de l'oligonucléotide ayant la séquence suivante : préalablement phosphorylé et hybridé sur lui-même de façon à introduire des sites *Eco*RI en bordure du bloc d'expression contenant le promoteur *GAL10*/*CYC1.* Le vecteur pTG10210 est ainsi obtenu.

### Stade 3 : construction du plasmide pTG10214

Le bloc d'expression contenant le promoteur *GAL10*/*CYC1* présent dans le vecteur pTG10210 a été ensuite introduit dans le vecteur navette *E. coli*-levure pTG10013 décrit par E. Degryse et al., 1995 et contenant le marqueur de sélection *URA3*-d.
Le vecteur pTG10013, après digestion avec l'enzyme de restriction *Eco*RI et traitement à la phosphatase alcaline, a été ligaturé dans le vecteur pTG10210 préparé au stade 1, préalablement digéré avec l'enzyme *Eco*RI. Le vecteur pTG10214 ainsi obtenu contient le bloc d'expression contenant le promoteur *GAL10*/*CYC1* dirigé vers le réplicon 2µ.

### Stade 3 : construction du plasmide pTG10274

Dans le plasmide pTG10214, le promoteur *GAL10*/*CYC1* a été ensuite échangé avec le promoteur *CYC1* par recombinaison homologue après excision du plasmide par traitement avec les enzymes de restriction *Cla*I et *Sal*I. Le vecteur d'expression pTG10031 décrit par E. Degryse et al., 1995 et contenant le promoteur *CYC1* a été digéré par les enzymes de restriction *Hind*III et *Fsp*I, puis recombiné avec le plasmide pTG10214 préparé au stade 2 et préalablement digéré avec les enzymes de restriction *Cla*I et *Sal*I, générant ainsi le plasmide pTG10274 (figure 16).

### Stade 4: construction du plasmide pTG10401

A partir du plasmide pTG10274 contenant le promoteur *CYC1* et du plasmide pTG10293 contenant la séquence codante pour le cytochrome P₄₅₀17α sous le contrôle du promoteur *TEF1,* un nouveau plasmide pTG10401 contenant la séquence codante pour le cytochrome P₄₅₀17α sous le contrôle du promoteur *TEF1* a été ensuite généré par recombinaison homologue. Le promoteur *CYC1* et une partie du réplicon d'*E. coli* ont été excisés du plasmide pTG10274, préparé au stade 3, par digestion avec les enzymes de restriction *Mlu*I et *Dra*I. Le plasmide pTG10293, préparé à l'exemple 5, a été digéré avec les enzymes de restriction *Hind*III et *Pvu*II, puis recombiné avec le plasmide pTG10274 digéré avec les enzymes de restriction *Mlu*I et *Dra*I générant le plasmide pTG10401 (figure 17).

### Stade 5 : construction du plasmide pTG10403

L'ADNc codant pour la 3β-HSD humaine de type II a été ensuite introduit dans le plasmide pTG10401 sous le contrôle du promoteur *CYC1.*

Dans un premier temps, le vecteur d'expression pTG10262 contenant l'ADNc codant pour la 3β-HSD humaine de type II, a été construit selon le schéma de la figure 18, à partir du vecteur de transfert pTG10095 préparé à l'exemple 3 et du vecteur de recombinaison pTG10257 contenant le réplicon de levure 2µ, un réplicon *d'E. coli,* la cassette d'expression de levure *CYC1*_{*prom*}*-PGK*_{*term*} et un marqueur de sélection *URA3-d.* Ce vecteur pTG10257 est identique au vecteur de recombinaison pTG10042 décrit par E. Degryse et al., 1995, à l'exception du site *Xba*I contenu dans la région 2µ et qui est remplacé par un marqueur *Xba*I° obtenu par remplissage du site *Xba*I naturel avec le fragment de Klenow puis religature.

Le bloc d'expression de la 3β-HSD humaine de type II a été excisé du vecteur de transfert pTG10095 par digestion avec l'enzyme de restriction *Not*I, puis introduit dans le vecteur de recombinaison pTG10257 préalablement digéré avec les enzymes de restriction *Sal*I et *Mlu*I, générant le vecteur d'expression pTG10262 (figure 19). Il faut noter que la recombinaison du bloc *GAL10*/*CYC1*-ADNc provenant du plamide pTG10095 dans le vecteur de recombinaison pTG10257 contenant le promoteur *CYC1* produit un vecteur d'expression contenant le bloc *CYC1*-ADNc.

Dans un deuxième temps, le vecteur d'expression pTG10262, préparé ci-dessus, a été digéré avec l'enzyme de restriction *Xmn*I. Le fragment obtenu contenant l'ADNC codant pour la 3β-HSD a été recombiné avec le fragment du plasmide pTG10401 préparé au stade 4 contenant l'ADNC codant pour le cytochrome P₄₅₀17α obtenu par digestion avec l'enzyme de restriction *Scal*I. Le plasmide pTG10403 (figure 20) ainsi obtenu contient deux blocs d'expression, l'un codant pour la 3β-HSDH sous le contrôle du promoteur *CYC1,* l'autre codant pour le cytochrome P₄₅₀17α sous le contrôle du promoteur *TEF1.*

### Stade 6 :construction du plasmide pTG10417

Enfin, dans le plasmide pTG10403 ci-dessus, le promoteur *TEF1* a été échangé avec le promoteur *CYC1.*

D'une part, le plasmide pTG10058 décrit à l'exemple 5 a été digéré avec l'enzyme de restriction *Pvu*II, ce qui permet de libérer une partie de la séquence codante pour le cytochrome P₄₅₀17α associée avec le promoteur *CYC1* ainsi que la majeure partie du réplicon *d'E. coli.* D'autre part, une partie du réplicon *d'E.* coli a été éliminée du plasmide pTG10403 préparé au stade 5 par digestion avec l'enzyme de restriction *Dra*I. Par recombinaison des deux plasmides préalablement ainsi digérés, le plasmide pTG10417 est finalement obtenu.

Le plasmide pTG10417 contient le réplicon de levure 2µ, le marqueur de sélection *URA3*-d et les deux blocs d'expression, l'un codant pour la 3β-HSD humaine, l'autre codant pour le cytochrome P₄₅₀17α d'origine bovine, tous les deux sous le contrôle du promoteur de levure *CYC1* (Figure 21).

Le plasmide pTG10417 a été ensuite transformé respectivement dans la souche parente FY1679 ou dans la souche TGY186 (*atf2-Δ: : TEF1*_{*prom*}/*PGK*_{*term*}*)* préparée à l'exemple 4.

Les transformants ont été isolés sur un milieu YNB (Difco) gélosé, contenant 0,5 % de glucose, enrichi en tryptophane, histidine et leucine (100 *µ*g/ml chacun).

Les colonies ainsi obtenues ont été ensuite précultivées pendant 24 heures à 28°C dans le milieu YNB (Difco) contenant 0,5 % de glucose et 0,1 % de casaminoacides, puis diluées à A600=0,1 et supplémentées en prégnénolone à 100 µg/ml, soit dans le même milieu frais (milieu 1), soit dans le milieu YNB (Difco) contenant 0,1 % de glucose, 2 % de glycérol et 0,2 % de casaminoacides (milieu 2). Après 48 heures de croissance et de bioconversion, les stéroides ont été extraits et mesurés par RP-HPLC comme indiqué à l'exemple 1 en utilisant les standards de prégnénolone, 17α-hydroxyprégnénolone, progestérone et 17α-hydroxyprogestérone. Les résultats obtenus exprimés en µg/ml sont montrés dans le tableau 5 (milieu 1) et le tableau 6 (milieu 2) suivants :

**Tableau 5**

| **Souche** | **FY1679/pTG10417** | **TGY186/pTG10417** |
|---|---|---|
| Prégnénolone | 0 | 58,1 |
| Prégnénolone acétate | 87,3 | 0 |
| 17α-hydroxyprégnénolone | 0 | 3,1 |
| 17α-hydroxyprégnénolone acétate | 0 | 0 |
| Progestérone | 10,6 | 1,4 |
| 17α-hydroxyprogestérone | 1,3 | 23,4 |

**Tableau 6**

| **Souche** | **FY1679/pTG10417** | **TGY186/pTG10417** |
|---|---|---|
| Prégnénolone | 0 | 54,0 |
| Prégnénolone acétate | 52,1 | 0 |
| 17α-hydroxyprégnénolone | 0 | 3,4 |
| 17α-hydroxyprégnénolone acétate | 12,8 | 0 |
| Progestérone | 0 | 1,4 |
| 17α-hydroxyprogestérone | 8,6 | 31,4 |

Ces résultats montrent que la bioconversion dans la souche sauvage (FY) transformée par le plasmide pTG10417 résulte dans l'accumulation de prégnénolone acétate et de 17α-hydroxyprégnénolone acétate qui ne sont pas transformés ultérieurement par les enzymes 3β-HSDH ou P₄₅₀17α et que le bilan de bioconversion est inférieur à celui observé avec le mutant *atf2* (TGY186) transformé.

Un échantillon de la souche transformée TGY186/pTG10417 a été déposé à la Collection Nationale de Cultures de Micro-organismes (CNCM) INSTITUT PASTEUR, 25, Rue du Docteur Roux 75724 PARIS CEDEX 15 FRANCE, le 20 janvier 1999 sous le numéro I-2118.

### Références bibliographiques

- Achstetter T., Nguyen-Juilleret M., Findeli A., Merkamm M. and Lemoine Y. (1992), Gene **110**, 25-31.
- Amberg D.C., Botstein D. and Beasley E.M., (1995), Yeast **11** : 1275-1280.
- Boeke Jef D., Lacroute F. and Fink G R., (1984) Mol Gen Genet 197 : 345-346.
- Cauet G., Dumas B., Degryse E., Spagnoli R. and Achstetter T. (1994) in Cytochrome P-450 biochemistry, biophysics and molecular biology (Lechner M.C., ed.) pp. 583-586, John Libbey Eurotext.
- Cherry J.M., Adler C., Ball C., Dwight S., Chervitz S., Jia Y., Juvik G., Roe T., Weng S. and Botstein D., "*Saccharomyces* Genome Database" http://genome-www.stanford. edu/*Saccharomyces*/
- Degryse E., J. Biotech. 39 (1995) 181-187.
- Degryse E., Dumas B., Dietrich M., Laruelle L. and Achstetter T. (1995). Yeast **11** : 629-640.
- Degryse E. (1996), Gene 170, 45-50.
- Dumas B., Cauet G., Degryse E., Spagnoli R. & Achtetter T. (1994). Cytochrome P450.8^{th} International Conférence. Ed. M. C. Lechner. John Libbey Eurotext, Paris pp. 527-530.
- Fiechter A., Fuhrmann G.F. and Kâppeli O., (1981). Adv. Microb. Physiol. **22**, 123-183.
- Fujii T., Nagasawa N., Iwamatsu A., Bogaki T., Tamai Y. and Hamachi M. (1994) Appl. Environ. Microbiol. **60**, 2786-2792.
- Hanahan D., J. Mol. Biol. 166 (1983) 557-580.
- Hubacek J. and Glover S.W., (1970), J. Mol. Biol. **50** : 111-127.
- Ito H., Fukuda Y., Murata K. and Kimura A., 1983, Journal of Bacteriology, 163-168.
- Rhéaume E., Lachance Y., Zhao H.-F., Breton N., Dumont M., de Launoit Y., Trudel C., Luu-The V., Simard J. & Labrie F. (1991). Mol. Endocrinol. 5, 1147-1157.
- Rose M., Grisafi P. and Botstein D., Gene 29, 113-124 (1984).
- Rosenfeld J., Capdevielle J., Guillemot J.C. and Ferrara P. (1992) Anal. Biochem. **203**, 173-179.
- Sambrook J., Fritsch E.F. and Maniatis T., (1989). Molecular Cloning : A Laboratory Manual. Cold Spring Harbor University Press, 2nd edition, Cold Spring Harbor.
- Sherman F. (1991) Methods in Enzymology 194, 3-21.
- Simard J., Durocher F., Mébarki F., Turgeon C., Sanchez R., Labrie Y., Couet J., Trudel C., Rhéaume E., Morel Y., Luu-The V. And Labrie F. (1996) J. Endocrinol. **150,** 5189-5207
- Struhl K., Stinchomb DT., Scherer S. and Davis RW., 1979, Proc. Natl. Acad. Sci. USA Vol. 76, N°3, 1035-1039.
- Thierry A., Fairhead C. and Dujon B., Yeast ; Vol. 6 : 521-534 (1990).
- Zuber MX., Simpson ER. and Waterman MR., (1986a) Science 234, 1258-1261.

## Revendications

1. Souche de levure modifiée dans laquelle l'activité acétyl-CoA prégnénolone acétyltransférase (APAT) est éliminée par altération du gène *ATF2* de *S. cerevisiae* ou un gène qui code pour une proteïne ayant l'activité APAT et ayant une identité de séquence d'environ 60% et plus avec la séquence de la proteïne YGR177C.

2. Souche de levure modifiée selon la revendication 1 dans laquelle le gène altéré est le gène *ATF2* de *S. cerevisiae.*

3. Souche de levure modifiée selon la revendication 2 dans laquelle le gène *ATF2* est altéré par insertion d'une séquence d'ADN ayant au moins un nucléotide.

4. Souche de levure modifiée selon la revendication 3 dans laquelle le gène *ATF2* est altéré par insertion du gène de sélection *URA3* ou du bloc d'expression de levure constitué du promoteur *TEF1* et du terminateur *PGK*.

5. Souche de levure modifiée selon la revendication 4 dans laquelle le gène ATF2 est altéré par insertion du gène de sélection *URA3.*

6. Souches de *S. cerevisiae* modifiées selon la revendication 5 dénommées TGY156 et déposée à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1977.

7. Souche de *S. cerevisiae* modifiée selon la revendication 5 énommée TGY158 et déposée à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1976.

8. Souche de levure modifiée selon la revendication 4 dans laquelle le gène *ATF2* est altéré par insertion du bloc d'expression de levure constitué du promoteur *TEF1* et du terminateur *PGK.*

9. Souche de levure transformée dans laquelle l'activité acétyl-CoA prégnénolone acétyltransférase (APAT) est éliminée par altération du gène ATF₂ de S Cerevisae ou un gène qui code pour une proteine ayant l'activité APAT et ayant une identité de sequence d'environ 60 % et plus avec la séquence de la proteïne YGR177C et exprimant au moins l'une des enzymes de la voie de biosynthèse de l'hydrocortisone à partir du cholestérol choisie parmi :
- l'enzyme de clivage de la chaîne latérale du cholestérol (P₄₅₀SCC),
- la 3β-hydroxy-delta⁵-stéroïde déshydrogénase/delta⁵-delta⁴-stéroide isomérase (3β-HSD) et
- la 17α-stéroïde hydroxylase (P₄₅₀17α).

10. Souche de levure transformée selon la revendication 9 dans laquelle le gène altéré est le gène *ATF2* de *S. cerevisiae* ou un homologue de celui-ci.

11. Souche de levure transformée selon la revendication 10 dans laquelle le gène altéré est le gène *ATF2* de *S. cerevisiae.*

12. Souche de levure transformée selon la revendication 11 dans laquelle le gène *ATF2* est altéré par insertion d'une séquence d'ADN ayant au moins un nucléotide.

13. Souche de levure transformée selon la revendication 11 dans laquelle le gène *ATF2* est altéré par insertion du gène de sélection *URA3.*

14. Souche de levure transformée selon la revendication 13 et exprimant la 3β-HSD.

15. Souche de *S. cerevisiae* transformée selon la revendication 14, dénommée TGY158/pTG10862 et déposée à la Collection Nationale de Cultures de Microorganismes (CNCM) INSTITUT PASTEUR, 25, Rue du Docteur Roux 75724 PARIS CEDEX 15 FRANCE, le 2 février 1998 sous le numéro I-1978.

16. Souche de levure transformée selon la revendication 11 dans laquelle le gène *ATF2* est altéré par insertion du bloc d'expression de levure constitué du promoteur *TEF1* et du terminateur *PGK*.

17. Souche de levure transformée selon la revendication 16 et exprimant P₄₅₀17α.

18. Souche de *S. cerevisiae* transformée selon la revendication 17, dénommée TGY186/pTG10435 et déposée à la Collection Nationale de Cultures de Microorganismes (CNCM) INSTITUT PASTEUR, 25, Rue du Docteur Roux 75724 PARIS CEDEX 15 FRANCE, le 20 janvier 1999 sous le numéro I-2119.

19. Souche de levure transformée selon la revendication 16 et coexprimant la 3β-HSD et P₄₅₀17α.

20. Souche de *S. cerevisiae* transformée selon la revendication 19, dénommée TGY186/pTG10417 et déposée à la Collection Nationale de Cultures de Micro-organismes (CNCM) INSTITUT PASTEUR, 25, Rue du Docteur Roux 75724 PARIS CEDEX 15 FRANCE, le 20 janvier 1999 sous le numéro I-2118.

21. Procédé d'oxydation *in vivo* d'un substrat choisi parmi un stérol endogène, un stérol exogène ou un stéroide exogène dans lequel on utilise une souche de levure transformée selon l'une des revendications 9 à 20 que, soit l'on cultive seule lorsque la souche génère le stérol endogène, soit l'on incube avec le stérol ou le stéroide exogène et on isole éventuellement le composé oxydé obtenu.

22. Procédé d'oxydation *in vivo* selon la revendication 21 dans lequel le substrat est un 3β-hydroxystéroide et dans lequel on utilise une souche de levure transformée selon la revendication 14 et isole éventuellement le 3-oxo-delta⁴-stéroïde obtenu.

23. Procédé d'oxydation *in vivo* selon la revendication 22 dans lequel le 3β-hydroxystéroïde est choisi parmi la prégnénolone ou la 17α-hydroxyprégnénolone.

24. Procédé d'oxydation *in vivo* selon la revendication 21 dans lequel le substrat est un stéroïde et dans lequel on utilise une souche de levure transformée selon la revendication 17 et isole éventuellement le stéroïde 17α-hydroxylé obtenu.

25. Procédé d'oxydation *in vivo* selon la revendication 24 dans lequel le substrat stéroïde est la prégnénolone ou la progestérone.

26. Procédé d'oxydation *in vivo* selon la revendication 21 dans lequel le substrat est un 3β-hydroxystéroïde et dans lequel on utilise une souche de levure transformée selon la revendication 19 et isole éventuellement le 3-oxo-delta⁴-stéroïde 17α-hydroxylé obtenu.

27. Procédé d'oxydation *in vivo* selon la revendication 26 dans lequel le substrat stéroide est la prégnénolone.

## Patentansprüche

1. Modifizierter Hefestamm, bei dem die Acetyl-CoA-Pregnenolon-Acetyltransferase (APAT)-Aktivität durch Veränderung des Gens *ATF2* von *S. cerevisiae* oder eines Gens, das für ein Protein kodiert, welches APAT-Aktivität hat und mit der Sequenz des Proteins YGR177C eine Sequenzidentität von 60% und mehr hat, eliminiert ist.

2. Modifizierter Hefestamm gemäß Anspruch 1, bei dem das veränderte Gen das Gen *ATF2* von *S. cerevisiae* ist.

3. Modifizierter Hefestamm gemäß Anspruch 2, bei dem das Gen ATF2 durch Insertion einer DNA-Sequenz, die mindestens ein Nucleotid hat, verändert ist.

4. Modifizierter Hefestamm nach Anspruch 3, in dem das Gen *ATF2* durch Insertion des Selektionsgens *URA3* oder des Expressionsblocks von Hefe, der aus dem Promotor *TEF1* und dem Terminator PGK besteht, verändert ist.

5. Modifizierter Hefestamm nach Anspruch 4, bei dem das Gen *ATF2* durch Insertion des Selektionsgens *URA3* verändert ist.

6. Modifizierte Stämme von *S. cerevisiae* nach Anspruch 5, als TGY156 bezeichnet und bei der Collection Nationale de Cultures de Microorganismes (CNCM) INSTITUT PASTEUR, 25, Rue du Docteur Roux 75724 PARIS CEDEX 15, Frankreich, am 2. Februar 1998 unter der Nummer I-1977 hinterlegt.

7. Modifizierter Stamm von *S. cerevisiae* gemäß Anspruch 5, TGY158 genannt und bei der Collection Nationale de Cultures de Microorganismes (CNCM) INSTITUT PASTEUR, 25, Rue du Docteur Roux 75724 PARIS CEDEX 15, Frankreich, am 02. Februar 1998 unter der Nummer I-1976 hinterlegt.

8. Modifizierter Hefestamm nach Anspruch 4, bei dem das Gen *ATF2* durch Insertion des Expressionsblocks von Hefe verändert ist, welcher aus dem Promotor *TEF1* und dem Terminator *PGK* besteht.

9. Transformierter Hefestamm, bei dem die Acetyl-CoA-Pregnenolon-Acetyltransferase (APAT)-Aktivität durch Veränderung des Gens *ATF2* von *S. cerevisiae* oder eines Gens, das für ein Protein kodiert, welches APAT-Aktivität hat und mit der Sequenz des Proteins YGR177C eine Sequenzidentität von 60% und mehr hat, eliminiert ist und der mindestens eines der Enzyme des Biosynthesewegs von Hydrocortison, ausgehend von Cholesterin, exprimiert, das ausgewählt ist aus:
- dem Enzym zur Spaltung der Seitenkette des Cholesterins (P₄₅₀SCC),
- 3β-Hydroxy-delta⁵-steroid-Dehydrogenase/delta⁵-delta⁴-steroid-Isomerase (3β-HSD) und
- 17α-Steroidhydroxylase (P₄₅₀17α).

10. Transformierter Hefestamm nach Anspruch 9, bei dem das veränderte Gen das Gen *ATF2* von *S. cerevisiae* oder ein Homologes desselben ist.

11. Transformierter Hefestamm nach Anspruch 10, bei dem das veränderte Gen das Gen *ATF2* von *S. cerevisiae* ist.

12. Transformierter Hefestamm nach Anspruch 11, bei dem das Gen *ATF2* durch Insertion einer DNA-Sequenz, die mindestens ein Nucleotid hat, verändert ist.

13. Transformierter Hefestamm nach Anspruch 11, bei dem das Gen *ATF2* durch Insertion des Selektionsgens *URA3* verändert ist.

14. Transformierter Hefestamm nach Anspruch 13, der 3β-HSD exprimiert.

15. Transformierter *S. cerevisiae*-Stamm nach Anspruch 14, TGY158/pTG10862 genannt und bei der Collection Nationale de Cultures de Microorganismes (CNCM) INSTITUT PASTEUR, 25, Rue du Docteur Roux 75724 PARIS CEDEX 15, Frankreich, am 2. Februar 1998 unter der Nummer I-1978 hinterlegt.

16. Transformierter Hefestamm nach Anspruch 11, bei dem das Gen *ATF2* durch Insertion des Expressionsblocks von Hefe verändert ist, welcher aus dem Promotor *TEF1* und dem Terminator *PGK* besteht.

17. Transformierter Hefestamm nach Anspruch 16, der P₄₅₀17α exprimiert.

18. Transformierter S. cerevisiae-Stamm nach Anspruch 17, TGY186/pTG10435 genannt und bei der Collection Nationale de Cultures de Microorganismes (CNCM) INSTITUT PASTEUR, 25, Rue du Docteur Roux 75724 PARIS CEDEX 15, Frankreich, am 20. Januar 1999 unter der Nummer I-2119 hinterlegt.

19. Transformierter Hefestamm nach Anspruch 16, der 3β-HSD und P₄₅₀17α co-exprimiert.

20. Transformierter *S. cerevisiae*-Stamm nach Anspruch 19, TGY186/pTG10417 genannt und bei der Collection Nationale de Cultures de Microorganismes (CNCM) INSTITUT PASTEUR, 25, Rue du Docteur Roux 75724 PARIS CEDEX 15, Frankreich, am 20. Januar 1999 unter der Nummer I-2118 hinterlegt.

21. Verfahren zur *in vivo*-Oxidation eines Substrats, das aus einem endogenen Sterol, einem exogenen Sterol oder einem exogenen Steroid ausgewählt ist, wobei man einen transformierten Hefestamm nach einem der Ansprüche 9 bis 20 verwendet, den man entweder allein kultiviert, wenn der Stamm das endogene Sterol bildet, oder den man mit dem Sterol oder dem exogenen Steroid inkubiert und gegebenenfalls die erhaltene oxidierte Verbindung isoliert.

22. Verfahren zur *in vivo*-Oxidation nach Anspruch 21, wobei das Substrat ein 3β-Hydroxysteroid ist und wobei man einen transformierten Hefestamm nach Anspruch 14 verwendet und gegebenenfalls das erhaltene 3-Oxo-delta⁴-steroid isoliert.

23. Verfahren zur *in vivo*-Oxidation nach Anspruch 22, wobei das 3β-Hydroxysteroid unter Pregnenolon und 17α-Hydroxy-Pregnenolon ausgewählt wird.

24. Verfahren zur *in vivo*-Oxidation nach Anspruch 21, wobei das Substrat ein Steroid ist und wobei man einen transformierten Hefestamm nach Anspruch 17 verwendet und gegebenenfalls das erhaltene 17α-hydroxylierte Steroid isoliert.

25. Verfahren zur *in vivo*-Oxidation nach Anspruch 24, wobei das Steroidsubstrat Pregnenolon oder Progesteron ist.

26. Verfahren zur *in vivo*-Oxidation nach Anspruch 21, wobei das Substrat ein 3β-Hydroxysteroid ist und wobei man einen transformierten Hefestamm nach Anspruch 19 verwendet und gegebenenfalls das erhaltene 17α-hydroxylierte 3-Oxo-delta⁴-steroid isoliert.

27. Verfahren zur *in vivo*-Oxidation nach Anspruch 26, wobei das Steroidsubstrat Pregnenolon ist.

## Claims

1. Modified yeast strain in which the acetyl-CoA pregnenolone acetyltransferase (APAT) activity is eliminated by alteration of the *S. cerevisiae ATF2* gene or a gene which encodes a protein having APAT activity and having a sequence identity of approximately 60% or more with the sequence of the YGR177C protein.

2. Modified yeast strain according to Claim 1, in which the altered gene is the *S. cerevisiae ATF2* gene.

3. Modified yeast strain according to Claim 2, in which the *ATF2* gene is altered by insertion of a DNA sequence having at least one nucleotide.

4. Modified yeast strain according to Claim 3, in which the *ATF2* gene is altered by insertion of the URA3 selection gene or of the yeast expression block consisting of the *TEF1* promoter and of the PGK terminator.

5. Modified yeast strain according to Claim 4, in which the *ATF2* gene is altered by insertion of the URA3 selection gene.

6. Modified *S. cerevisiae* strains according to Claim 5, called TGY156 and deposited with the Collection Nationale de Cultures de Microorganismes (CNCM) [National Collection of Cultures and Microorganisms], INSTITUT PASTEUR, 25, Rue du Docteur Roux 75724 PARIS CEDEX 15 FRANCE, on 2 February 1998 under the number I-1977.

7. Modified *S. cerevisiae* strain according to Claim 5, called TGY158 and deposited with the Collection Nationale de Cultures de Microorganismes (CNCM), INSTITUT PASTEUR, 25, Rue du Docteur Roux 75724 PARIS CEDEX 15 FRANCE, on 2 February 1998 under the number I-1976.

8. Modified yeast strain according to Claim 4, in which the *ATF2* gene is altered by insertion of the yeast expression block consisting of the *TEF1* promoter and of the *PGK* terminator.

9. Transformed yeast strain in which the acetyl-CoA pregnenolone acetyltransferase (APAT) activity is eliminated by alteration of the S. *cerevisiae ATF2* gene or a gene which encodes a protein having APAT activity and having a sequence identity of approximately 60% or more with the sequence of the YGR177C protein, and which expresses at least one of the enzymes of the pathway for biosynthesis of hydrocortisone from cholesterol, chosen from:
- the cholesterol side-chain cleavage enzyme (P₄₅₀SCC),
- 3β-hyroxy-delta⁵-steroid dehydrogenase/delta⁵-delta⁴-steroid isomerase (3β-HSD), and
- 17α-steroid hydroxylase (P₄₅₀17α).

10. Transformed yeast strain according to Claim 9, in which the altered gene is the *S. cerevisiae ATF2* gene or a homologue thereof.

11. Transformed yeast strain according to Claim 10, in which the altered gene is the *S. cerevisiae ATF2* gene.

12. Transformed yeast strain according to Claim 11, in which the *ATF2* gene is altered by insertion of a DNA sequence having at least one nucleotide.

13. Transformed yeast strain according to Claim 11, in which the *ATF2* gene is altered by insertion of the *URA3* selection gene.

14. Transformed yeast strain according to Claim 13 and which expresses 3β-HSD.

15. Transformed *S. cerevisiae* strain according to Claim 14, called TGY158/pTG10862 and deposited with the Collection Nationale de Cultures de Microorganismes (CNCM), INSTITUT PASTEUR, 25, Rue du Docteur Roux 75724 PARIS CEDEX 15 FRANCE, on 2 February 1998 under the number I-1978.

16. Transformed yeast strain according to Claim 11, in which the *ATF2* gene is altered by insertion of the yeast expression block consisting of the *TEF1* promoter and of the *PGK* terminator.

17. Transformed yeast strain according to Claim 16 and which expresses P₄₅₀17α.

18. Transformed *S. cerevisiae* strain according to Claim 17, called TGY186/pTG10435 and deposited with the Collection Nationale de Cultures de Microorganismes (CNCM), INSTITUT PASTEUR, 25, Rue du Docteur Roux 75724 PARIS CEDEX 15 FRANCE, on 20 January 1999 under the number I-2119.

19. Transformed yeast strain according to Claim 16 and which coexpresses 3β-HSD and P₄₅₀17α.

20. Transformed *S. cerevisiae* strain according to Claim 19, called TGY186/pTG10417 and deposited with the Collection Nationale de Cultures de Microorganismes (CNCM), INSTITUT PASTEUR, 25, Rue du Docteur Roux 75724 PARIS CEDEX 15 FRANCE, on 20 January 1999 under the number I-2118.

21. Method of *in vivo* oxidation of a substrate chosen from an endogenous sterol and exogenous sterol or an exogenous steroid, in which use is made of a transformed yeast strain according to one of Claims 9 to 20, which is either cultured alone when the strain generates endogenous sterol or which is incubated with the exogenous sterol or the exogenous steroid, and the oxidized compound obtained is optionally isolated.

22. Method of *in vivo* oxidation according to Claim 21, in which the substrate is a 3β-hydroxysteroid, and in which use is made of a transformed yeast strain according to Claim 14 and the 3-oxo-delta⁴-steroid obtained is optionally isolated.

23. Method of *in vivo* oxidation according to Claim 22, in which the 3β-hydroxysteroid is chosen from pregnenolone and a 17α-hydroxypregnenolone.

24. Method of *in vivo* oxidation according to Claim 21, in which the substrate is a steroid, and in which use is made of a transformed yeast strain according to Claim 17 and the 17α-hydroxylated steroid obtained is optionally isolated.

25. Method of *in vivo* oxidation according to Claim 24, in which the steroid substrate is pregnenolone or progesterone.

26. Method of *in vivo* oxidation according to Claim 21, in which the substrate is a 3β-hydroxysteroid, and in which use is made of a transformed yeast strain according to Claim 19 and the 17α-hydroxylated 3-oxo-delta⁴-steroid obtained is optionally isolated.

27. Method of *in vivo* oxidation according to Claim 26, in which the steroid substrate is pregnenolone.


### LISTE DE SEQUENCES

<110> Hoechst Marion Roussel
<120> Souches de levure ayant le gène ATF2 interrompu et leurs applications.
<130> 2482 PCT SEQUENCES EN FRANCAIS
<140>
<141>
<160> 17
<170> PatentIn Ver. 2.1
<210> 1
<211> 50
<212> ADN
<213> Séquence artificielle
<220>
<223> OLIGONUCLEOTIDE
<400> 1
   aaaagtcgac aaaatggaag atatagaagg atacgaacca catatcactc 50
<210> 2
<211> 33
<212> ADN
<213> Séquence artificielle
<220>
<223> OLIGONUCLEOTIDE
<220>
<221> misc_feature
<222> Complement((1)..(33))
<400> 2
   atcaatctcc aattaggcct cttcggatta ccc 33
<210> 3
<211> 32
<212> ADN
<213> Séquence artificielle
<220>
<223> OLIGONUCLEOTIDE
<400> 3
   cattcgacat tcccgaaggt gacaatgaca ag 32
<210> 4
<211> 46
<212> ADN
<213> Séquence artificielle
<220>
<223> OLIGONUCLEOTIDE
<220>
<221> misc_feature
<222> Complement((1)..(46))
<400> 4
   aaaaacgcgt aactattaaa gcgacgcaaa ttcgccgatg gtttgg 46
<210> 5
<211> 79
<212> ADN
<213> Séquence artificielle
<220>
<223> OLIGONUCLEOTIDE
<400> 5
   gggtaatccg aagaggccta attggagatt gataagcttt tcaattcaat tcatcatttt 60
   ttttttattc ttttttttg 79
<210> 6
<211> 63
<212> ADN
<213> Séquence artificielle
<220>
<223> OLIGONUCLEOTIDE
<220>
<221> misc_feature
<222> Complement((1)..(63))
<400> 6
   cttgtcattg tcaccttcgg gaatgtcgaa tggggtaata actgatataa ttaaattgaa 60
   ctc 63
<210> 7
<211> 12
<212> ADN
<213> Séquence artificielle
<220>
<223> OLIGONUCLEOTIDE
<400> 7
   cacacgcgtg tg 12
<210> 8
<211> 52
<212> ADN
<213> Séquence artificielle
<220>
<223> OLIGONUCLEOTIDE
<400> 8
   ctctctgtcg acaaaatgga agatatagaa ggatacgaac cacatatcac tc 52
<210> 9
<211> 33
<212> ADN
<213> Séquence artificielle
<220>
<223> OLIGONUCLEOTIDE
<220>
<221> misc_feature
<222> Complement((1)..(33))
<400> 9
   atcaatctcc aattaggcct cttcggatta ccc 33
<210> 10
<211> 32
<212> ADN
<213> Séquence artificielle
<220>
<223> OLIGONUCLEOTIDE
<400> 10
   cattcgacat tcccgaaggt gacaatgaca ag 32
<210> 11
<211> 48
<212> ADN
<213> Séquence artificielle
<220>
<223> OLIGONUCLEOTIDE
<220>
<221> misc_feature
<222> Complement((1)..(48))
<400> 11
   aacaacacgc gtaactatta aagcgacgca aattcgccga tgctttgg 48
<210> 12
<211> 68
<212> ADN
<213> Séquence artificielle
<220>
<223> OLIGONUCLEOTIDE
<400> 12
   gggtaatccg aagaggccta attggagatt gatatcgatc acacaccata gcttcaaaat 60
   gtttctac 68
<210> 13
<211> 67
<212> ADN
<213> Séquence artificielle
<220>
<223> OLIGONUCLEOTIDE
<220>
<221> misc_feature
<222> Complement((1)..(67))
<400> 13
   cttgtcattg tcaccttcgg gaatgtcgaa gcttcgaaac gcagaatttt cgagttatta 60
   aacttaa 67
<210> 14
<211> 10
<212> ADN
<213> Séquence artificielle
<220>
<223> OLIGONUCLEOTIDE
<400> 14
   gtcgaagctt 10
<210> 15
<211> 15
<212> ADN
<213> Séquence artificielle
<220>
<223> OLIGONUCLEOTIDE
<400> 15
   tcgacggacg cgtgg 15
<210> 16
<211> 15
<212> ADN
<213> Séquence artificielle
<220>
<223> OLIGONUCLEOTIDE
<220>
<221> misc_feature
<222> Complement ((1) .. (15))
<400> 16
   tcgaccacgc gtccg                                                    15
<210> 17
<211> 12
<212> ADN
<213> Séquence artificielle
<220>
<223> OLIGONUCLEOTIDE
<400> 17
   cccgaattcg gg                                                       12